(19) Europäisches Patentamt
European Patent Office
Office européen des brevets

(11) Veröffentlichungsnummer: **0 224 181 B1**

(12) # EUROPÄISCHE PATENTSCHRIFT

(45) Veröffentlichungstag der Patentschrift: **04.09.91**

(51) Int. Cl.5: **C07D 498/10**, C08G 59/14, C08K 5/353, C08L 23/00, C08L 25/00, C09D 7/12

(21) Anmeldenummer: **86115961.4**

(22) Anmeldetag: **18.11.86**

(54) **Substituierte Diazaoxaspirodecane, ihre Herstellung und ihre Verwendung als Stabilisatoren für Polymere.**

(30) Priorität: **26.11.85 DE 3541665**

(43) Veröffentlichungstag der Anmeldung:
**03.06.87 Patentblatt 87/23**

(45) Bekanntmachung des Hinweises auf die Patenterteilung:
**04.09.91 Patentblatt 91/36**

(84) Benannte Vertragsstaaten:
**AT BE CH DE FR GB IT LI NL SE**

(56) Entgegenhaltungen:
**EP-A- 0 057 885**
**EP-A- 0 079 850**
**CH-A- 635 853**
**CH-A- 648 036**
**US-A- 4 371 644**

(73) Patentinhaber: **HOECHST AKTIENGESELLSCHAFT**
**Postfach 80 03 20**
**W-6230 Frankfurt am Main 80(DE)**

(72) Erfinder: **Ertl, Josef, Dr.**
**Eichenstrasse 14**
**W-8857 Wertingen(DE)**

**EP 0 224 181 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue sterisch gehinderte Aminlichtstabilisatoren, die synthetische Polymere gegen die Einwirkung von Licht, Wärme und Sauerstoff schützen.

Die DE-OS 31 04 294 = EP-A 57 885 umfaßt unter anderem auch Polyalkylpiperidin-Lichtschutzmittel der Formel

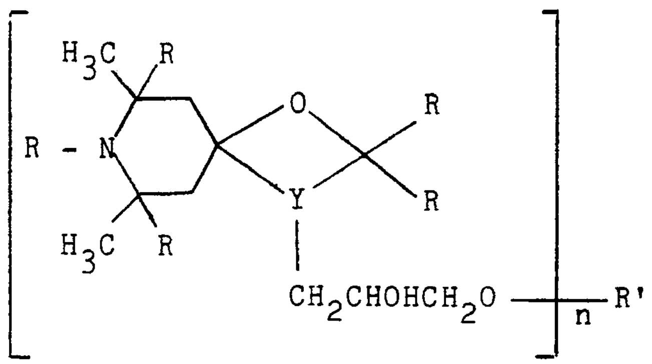

wobei

| | |
|---|---|
| n | 1 bis 3 und |
| R' bei n = 1 | für Wasserstoff, Phenyl, für $C_1$- bis $C_{30}$-Alkyl, für eine $C_1$- bis $C_{18}$-alkylsubstituierte, $C_5$- bis $C_6$-cycloalkylsubstituierte oder phenylsubstituierte Acyl- oder Carbamoylgruppe, deren |

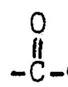-Gruppe

an den Sauerstoff gebunden ist, steht,

| | |
|---|---|
| R' bei n = 2 | für $C_2$- bis $C_{18}$-Alkylen, für unsubstituiertes oder durch bis zu zwei $C_1$- bis $C_4$-Alkylgruppen substituiertes Phenylen, für a, -Dicarboxy-$C_1$ bis $C_8$-Alkylen, für einen Dicarboxy-$C_6$-Ring, für $C_7$- bis $C_{14}$-Aralkylen steht und |
| R' bei n = 3 | für einen Isocyanursäurerest oder einen |

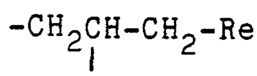-Rest

steht.

Die vorliegende Erfindung betrifft nun Verbindungen der Formel (I)

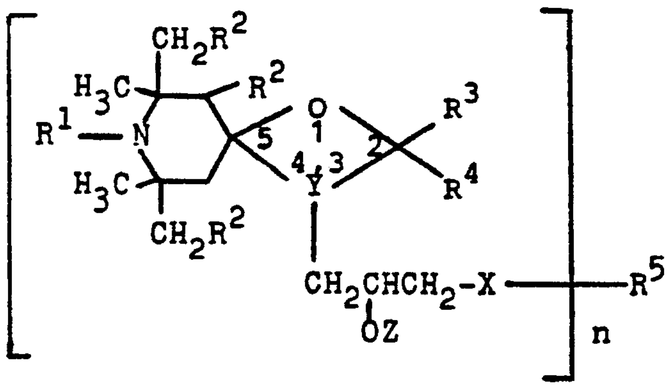

(I),

worin

n gleich 1 bis 20, Vorzugsweise 2 bis 10, insbesondere 2 bis 5 ist,

$R^1$ Wasserstoff, $C_1$- bis $C_4$-Alkyl, Benzyl, Allyl, $C_2$- bis $C_{30}$-Alkanoyl, $C_3$- bis $C_{20}$-Alkenoyl, $C_7$- bis $C_{11}$-Aroyl, $C_8$- bis $C_{14}$-Arylalkanoyl oder $C_8$- bis $C_{20}$-Alkylaryl, Vorzugsweise Wasserstoff, $C_1$- bis $C_4$-Alkyl, $C_2$- bis $C_{30}$-Alkanoyl, insbesondere Wasserstoff oder Acyl, ganz besonders bevorzugt Wasserstoff ist,

$R^2$ Wasserstoff oder $C_1$- bis $C_4$-Alkyl, Vorzugsweise Wasserstoff ist,

$R^3$ und $R^4$ gleich oder verschieden sind und Wasserstoff, $C_1$- bis $C_{18}$-, vorzugsweise $C_1$ bis $C_{13}$- und insbesondere $C_1$- bis $C_9$-Alkyl, unsubstituiertes oder durch Chlor oder $C_1$- bis $C_4$-Alkyl substituiertes Phenyl, unsubstituiertes oder durch $C_1$- bis $C_4$-Alkyl substituiertes $C_7$- bis $C_{14}$-Aralkyl, vorzugsweise $C_7$- bis $C_9$-Phenylalkyl, oder auch zusammen mit dem diese Reste bindenden Kohlenstoffatom einen unsubstituierten oder durch bis zu vier $C_1$- bis $C_4$-Alkylgruppen substituierten $C_5$- bis $C_{12}$-Cycloalkyl- oder Piperidinring bedeuten,

$R^5$ bei n = 1 einen mit 1 bis 4 $C_1$- bis $C_4$-Alkylgruppen substituierten Piperindinring, vorzugsweise 2,2,6,6-Tetramethyl-4-piperidinyl, bedeutet,

$R^5$ bei n = 2 Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$- bis $C_4$-Alkylreste substituiert sein können, einen Rest

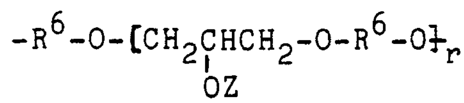

mit

r = 1 bis 20 und $R^6$ gleich $C_2$- bis $C_{12}$-Alkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$- bis $C_4$-Alkylreste substituiert sein können, bedeutet,

$R^5$ bei n = 3 den Rest eines trifunktionellen Amins, den trifunktionellen Rest eines aliphatischen Alkohols, der noch weitere Hydroxylgruppen enthält, vorzugsweise

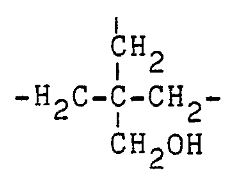

,

den trifunktionellen Rest eines Novolaks auf Basis Phenol, Kresol, Bisphenol-F oder Bisphenol-A bedeutet,

$R^5$ bei n = 4 den tetrafunktionellen Rest eines aliphatischen Alkohols oder Amins, vorzugsweise den Pentaerythritylrest oder eines Novolaks auf Basis Phenol, Kresol, Bisphenol-F oder Bisphenol-A oder den Rest

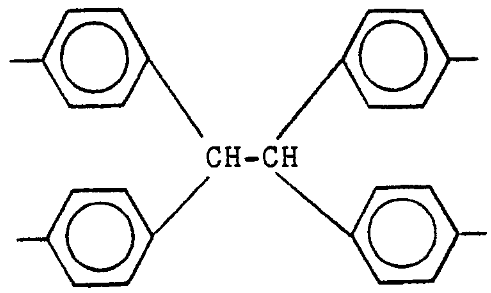

bedeutet,

$R^5$ bei n>4 einen Polyfunktionellen Rest eines Polyols, eines Polyamins, eines Novolaks auf Basis Phenol, Kresol, Bisphenol-F oder Bisphenol-A bedeutet,

Y 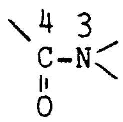 oder 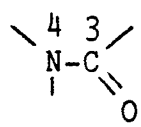 bedeutet und

die Ringpositionen 3, 4 in Formel (I) einnimmt,

X -O- oder 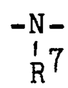 darstellt, wobei

$R^7$ Wasserstoff, $C_1$- bis $C_{30}$-Alkyl, ein durch 1 bis 4 $C_1$- bis $C_4$-Alkylgruppen substituierter Piperidinring oder ein Rest der Formel (II)

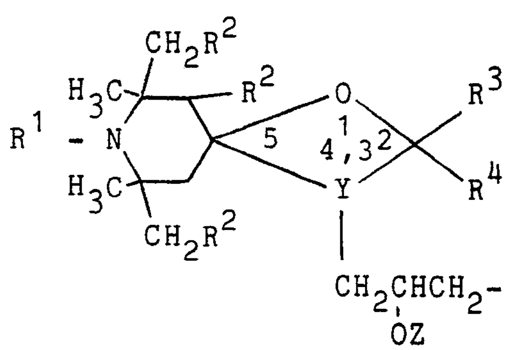

(II)

worin $R^1$ R2 $R^3$, $R^4$ und Y die oben angegebenen Bedeutungen haben, ist,

z Wasserstoff oder ein Rest der Formel (III)

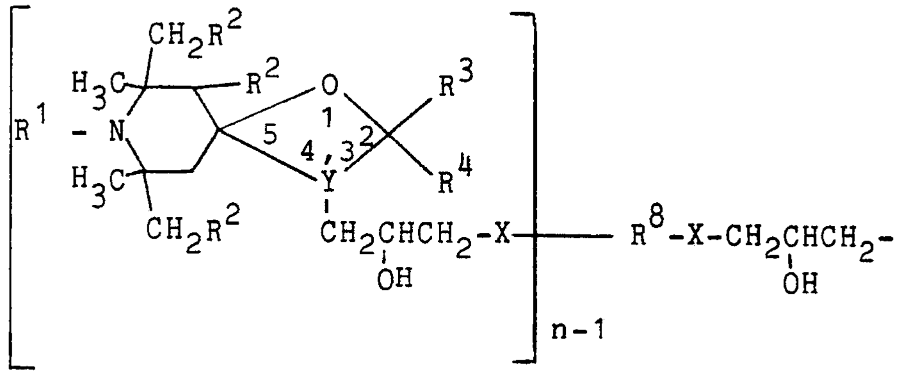

(III)

worin $R^1$ R2 $R^3$, $R^4$, X, Y, Z und n die oben angegebenen Bedeutungen haben, oder ein Rest der Formel (II) ist.

$R^8$ hat die Bedeutung von $R^5$ und bedeutet zusätzlich noch

bei n = 1 Wasserstoff, $C_1$- bis $C_{30}$-Alkyl, Aryl, Aralkyl, Alkylaryl oder ein durch 1 bis 4 $C_1$- bis $C_4$-Alkylgruppen substituierter Piperidinring und

bei n = 2 $C_2$- bis $C_{18}$-Alkylen oder Phenylen, wobei dann

$$X \text{ nur } -\underset{\substack{|\\R^7}}{N}-$$

sein darf.

Zusätzlich steht die Gruppierung

$$[-\underset{\substack{|\\R^7}}{N}-]_n R^5$$

aus Formel (I)

bei n = 2 für

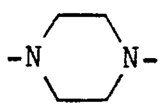 oder $R^7-\underset{|}{N}-(CH_2)_s-\underset{|}{N}-R^7$ und

bei n ≧ 2 für ein Polyamin der Formeln

$$R^7-\underset{|}{N}-(CH_2)_s-\underset{|}{N}-(CH_2)_t\underset{|}{N}-R^7$$

$$\text{oder } R^7-\underset{|}{N}-(CH_2)_s\underset{|}{N}-(CH_2)_t-\underset{|}{N}-(CH_2)_s\underset{|}{N}-R^7 ,$$

wobei $R^7$ die oben angegebene Bedeutung hat und s und t ganze Zahlen von 2 bis 4 sind.

Die neuen Verbindungen der Formel I werden aus Diazaoxaspirodecanen der Formel (IVa) oder (IVb)

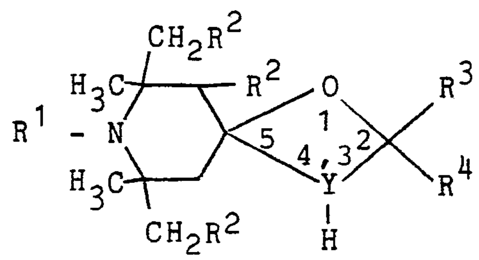

(IVa)

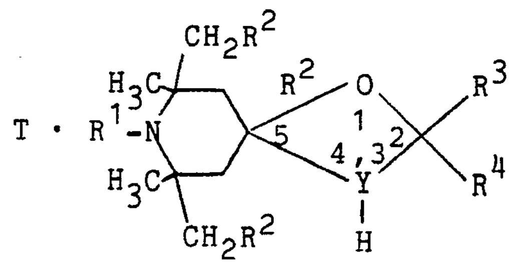

(IVb)

worin $R^1$, $R^2$, $R^3$, $R^4$ und Y die oben angegebenen Bedeutungen haben und T eine nicht oxidierende Mineralsäure oder eine aliphatische oder aromatische Sulfon- oder Phosphonsäure, eine aliphatische Mono-, Di- oder Polycarbonsäure oder eine aromatische Mono- oder Dicarbonsäure ist,
und

A) Epoxiden der Formel (Va) bzw. (Vb)

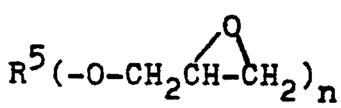

(Va)

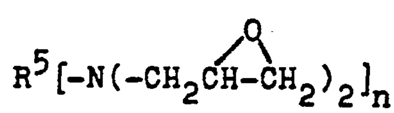

(Vb)

worin $R^5$ und n die oben angegebenen Bedeutungen haben, in einem inerten Lösemittel in Gegenwart einer Base und gegebenenfalls eines Phasentransferkatalysators bei 40 bis 180° C hergestellt, oder
B) die Verbindungen der Formel (IV) werden mit Epichlorhydrin zu einer Verbindung der Formel (VI)

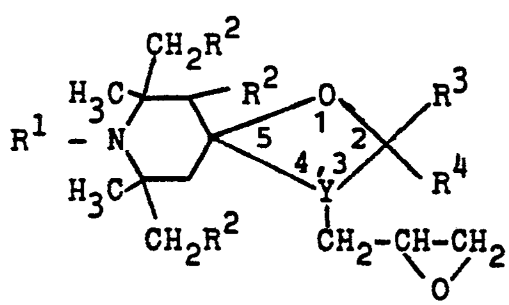

(VI)

und diese Verbindung der Formel (VI) dann mit einem Alkohol der Formel (VII)

$R^5(-OH)_n$ (VII)

oder einem Amin der Formel (VIII)

$R^5(-OH)_n$ (VII)

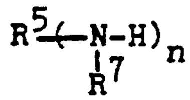

(VIII)

worin $R^1$, $R^2$, $R^3$, $R^4$, $R^5$, $R^7$, Y und n die oben angegebenen Bedeutungen haben, oder einem Polyamin der Formel (IX)

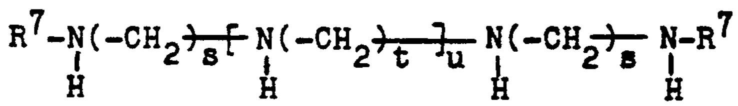

(IX)

worin $R^7$, s und t die oben angegebenen Bedeutungen haben und u eine ganze Zahl von 0 bis 3 ist, umgesetzt.

Beim Reaktionsweg A sowie bei der Umsetzung von Verbindungen der Formel (IV) mit Epichlorhydrin (Weg B, Teil 1) arbeitet man in einem inerten organischen Lösemittel, wie z.B. Toluol oder Xylol. Die Reaktion wird in Gegenwart der 0,2-bis 1,5-fach äquivalenten Menge einer Base, bezogen auf die Verbindung IVa, bzw. mit der 1,2- bis 2,5-fach äquivalenten Menge einer Base bei Einsatz einer Verbindung IVb, vorzugsweise mit der 0,3- bis 1,2-fach bzw. 1,3- bis 2,2-fach äquivalenten Menge Natriumhydroxid oder Kaliumhydroxid durchgeführt. Gegebenenfalls setzt man Phasentransferkatalysatoren zu. Die Verbindungen IV werden dabei in der 0,9-bis 1,1-fach äquivalenten Menge, vorzugsweise in der 0,95-bis 1,05-fach äquivalenten, besonders bevorzugt im Äquivalenz-Verhältnis 1:1, bezogen auf die Epoxidgruppen in den Verbindungen V, eingesetzt. Die Umsetzung erfolgt bei 40 - 180 °C, bevorzugt bei 50 - 150 °C, besonders bevorzugt bei 80 - 110 °C.

Die Umsetzung der 1-diazaoxaspirodecansubstituierten 2,3-Epoxipropyl-VerbindungVI beim Weg B mit Alkoholen bzw. Aminen erfolgt nach prinzipiell bekannten Methoden.

Die Ausgangsmaterialien der Formel (IVa) bzw. (IVb) sind an sich bereits gute Stabilisatoren, befriedigen jedoch hinsichtlich der Verträglichkeit mit dem zu stabilisierenden Polymeren und der Flüchtigkeit nicht in vollem Maße. Die erfindungsgemäßen Stabilisatoren weisen diese Nachteile nicht auf und zeigen überraschenderweise auch eine deutlich bessere antioxidative und lichtstabilisierende Wirkung.

Gegenüber den Verbindungen der DE-OS 31 04 294 besitzen die neuen Stabilisatoren bessere Eigenschaften, vor allem bessere antioxidative Wirkung und deutlich bessere Lichtschutzwirkung, was nicht erwartet werden konnte.

Geeignete Verbindungen der Formel (IVa) sind beispielsweise:

1. 2-Butyl-7,7,9,9-tetramethyl-1-oxa-3,8-diaza-4-oxo-spiro-[4,5]-decan
2. 2-iso-Butyl-7,7,9,9,-tetramethyl-1-oxa-3,8-diaza-4-oxo-spiro-[4,5]-decan
3. 2-Pentyl-7,7,9,9-tetramethyl-1-oxa-3,8-diaza-4-oxo-spiro-[4,5]-decan
4. 2-iso-Pentyl-7,7,9,9-tetramethyl-1-oxa-3,8-diaza-4-oxo-spiro-[4,5]-decan
5. 2-Hexyl-7,7,9,9-tetramethyl-1-oxa-3,8-diaza-4-oxo-spiro-[4,5]-decan
6. 2-Heptyl-7,7,9,9-tetramethyl-1-oxa-3,8-diaza-4-oxo-spiro-[4,5]-decan
7. 2-iso-Heptyl-7,7,9,9-tetramethyl-1-oxa-3,8-diaza-4-oxo-spiro-[4,5]-decan
8. 2-Nonyl-7,7,9,9-tetramethyl-1-oxa-3,8-diaza-4-oxo-spiro-[4,5]-decan
9. 2-iso-Nonyl-7,7,9,9-tetramethyl-1-oxa-3,8-diaza-4-oxo-spiro-[4,5]-decan
10. 2-Undecyl-7,7,9,9-tetramethyl-1-oxa-3,8-diaza-4-oxo-spiro-[4,5]-decan
11. 2-Phenyl-7,7,9,9-tetramethyl-1-oxa-3,8-diaza-4-oxo-spiro-[4,5]-decan
12. 2-(4-Chlor-phenyl)-7,7,9,9-tetramethyl-1-oxa-3,8-diaza-4-oxo-spiro-[4,5]-decan
13. 2-Ethyl-2,7,7,9,9-pentamethyl-1-oxa-3,8-diaza-4-oxo-spiro-[4,5]-decan
14. 2-Propyl-2,7,7,9,9-pentamethyl-1-oxa-3,8-diaza-4-oxo-spiro-[4,5]-decan
15. 2-iso-Propyl-2,7,7,9,9-pentamethyl-1-oxa-3,8-diaza-4-oxo-spiro-[4,5]-decan
16. 2-Butyl-2,7,7,9,9-pentamethyl-1-oxa-3,8-diaza-4-oxo-spiro-[4,5]-decan
17. 2-iso-Butyl-2,7,7,9,9-pentamethyl-1-oxa-3,8-diaza-4-oxo-spiro-[4,5]-decan
18. 2-Pentyl-2,7,7,9,9-pentamethyl-1-oxa-3,8-diaza-4-oxo-spiro-[4,5]-decan
19. 2-Hexyl-2,7,7,9,9-pentamethyl-1-oxa-3,8-diaza-4-oxo-spiro-[4,5]-decan
20. 2-Nonyl-2,7,7,9,9-pentamethyl-1-oxa-3,8-diaza-4-oxo-spiro-[4,5]-decan
21. 2,2,7,7,9,9-Hexamethyl-1-oxa-3,8-diaza-4-oxo-spiro-[4,5]-decan

22. 2,2,7,7,8,9,9-Heptamethyl-1-oxa-3,8-diaza-4-oxo-spiro-[4,5]-decan
23. 2,2-Diethyl-7,7,9,9-tetramethyl-1-oxa-3,8-diaza-4-oxo-spiro-[4,5]-decan
24. 2,2-Dipropyl-7,7,9,9-tetramethyl-1-oxa-3,8-diaza-4-oxo-spiro-[4,5]-decan
25. 2,2-Dibutyl-7,7,9,9-tetramethyl-1-oxa-3,8-diaza-4-oxo-spiro-[4,5]-decan
26. 2-Ethyl-2-pentyl-7,7,9,9-tetramethyl-1-oxa-3,8-diaza-4-oxo-spiro-[4,5]-decan
27. 2,2-Dibenzyl-7,7,9,9-tetramethyl-1-oxa-3,8-diaza-4-oxo-spiro-[4,5]-decan
28. 2,2,4,4-Tetramethyl-7-oxa-3,13-diaza-14-oxo-dispiro-[5,1,4,2]-tetradecan
29. 2,2,4,4-Tetramethyl-7-oxa-3,14-diaza-15-oxo-dispiro-[5,1,5,2]-pentadecan
30. 2,2,4,4-Tetramethyl-7-oxa-3,20-diaza-21-oxo-dispiro-[5,1,11,2]-heneicosan
31. 2,2,7,7,9,9-Hexamethyl-1-oxa-3,8-diaza-4-oxo-8-acetyl-spiro-[4,5]-decan
32. 2,2,4,4-Tetramethyl-7-oxa-3,14-diaza-15-oxo-3-acetyl-dispiro-[5,1,5,2]-pentadecan
33. 2,2,4,4-Tetramethyl-7-oxa-3,20-diaza-21-oxo-3-acetyl-dispiro-[5,1,11,2]-heneicosan
34. 2,7,7,9,9-Pentamethyl-1-oxa-4,8-diaza-3-oxo-spiro-[4,5]-decan
35. 2-Ethyl-7,7,9,9-tetramethyl-1-oxa-4,8-diaza-3-oxo-spiro-[4,5]-decan
36. 2-Propyl-7,7,9,9-tetramethyl-1-oxa-4,8-diaza-3-oxo-spiro-[4,5]-decan
37. 2-Butyl-7,7,9,9-tetramethyl-1-oxa-4,8-diaza-3-oxo-spiro-[4,5]-decan
38. 2-iso-Butyl-7,7,9,9-tetramethyl-1-oxa-4,8-diaza-3-oxo-spiro-[4,5]-decan
39. 2-Pentyl-7,7,9,9-tetramethyl-1-oxa-4,8-diaza-3-oxo-spiro-[4,5]-decan
40. 2-iso-Pentyl-7,7,9,9-tetramethyl-1-oxa-4,8-diaza-3-oxo-spiro-[4,5]-decan
41. 2-iso-Heptyl-7,7,9,9-tetramethyl-1-oxa-4,8-diaza-3-oxo-spiro-[4,5]-decan
42. 2-Phenyl-7,7,9,9-tetramethyl-1-oxa-4,8-diaza-3-oxo-spiro-[4,5]-decan
43. 2,2,7,7,9,9-Hexamethyl-1-oxa-4,8-diaza-3-oxo-spiro-[4,5]-decan
44. 2,2,7,7,8,9,9-Heptamethyl-1-oxa-4,8-diaza-3-oxo-spiro-[4,5]-decan
45. 2,2-Diethyl-7,7,9,9-tetramethyl-1-oxa-4,8-diaza-3-oxo-spiro-[4,5]-decan
46. 2,2,-Diethyl-7,7,8,9,9-pentamethyl-1-oxa-4,8-diaza-3-oxo-spiro-[4,5]-decan
47. 2,2,-Dipropyl-7,7,9,9-tetramethyl-1-oxa-4,8-diaza-3-oxo-spiro-[4,5]-decan
48. 2,2,-Dibutyl-7,7,9,9-tetramethyl-1-oxa-4,8-diaza-3-oxo-spiro-[4,5]-decan
49. 2,2-Dipentyl-7,7,9,9-tetramethyl-1-oxa-4,8-diaza-3-oxo-spiro-[4,5]-decan
50. 2-Ethyl-2,7,7,9,9-pentamethyl-1-oxa-4,8-diaza-3-oxo-spiro-[4,5]-decan
51. 2-Propyl-2,7,7,9,9-pentamethyl-1-oxa-4,8-diaza-3-oxo-spiro-[4,5]-decan
52. 2-iso-Propyl-2,7,7,9,9-pentamethyl-1-oxa-4,8-diaza-3-oxo-spiro-[4,5]-decan
53. 2-Butyl-2,7,7,9,9-pentamethyl-1-oxa-4,8-diaza-3-oxo-spiro-[4,5]-decan
54. 2-iso-Butyl-2,7,7,9,9-pentamethyl-1-oxa-4,8-diaza-3-oxo-spiro-[4,5]-decan
55. 2-Pentyl-2,7,7,9,9-pentamethyl-1-oxa-4,8-diaza-3-oxo-spiro-[4,5]-decan
56. 2-iso-Pentyl-2,7,7,9,9-pentamethyl-1-oxa-4,8-diaza-3-oxo-spiro-[4,5]-decan
57. 2-Hexyl-2,7,7,9,9-pentamethyl-1-oxa-4,8-diaza-3-oxo-spiro-[4,5]-decan
58. 2-Heptyl-2,7,7,9,9-pentamethyl-1-oxa-4,8-diaza-3-oxo-spiro-[4,5]-decan
59. 2-Nonyl-2,7,7,9,9-pentamethyl-1-oxa-4,8-diaza-3-oxo-spiro-[4,5]-decan
60. 2-Undecyl-2,7,7,9,9-pentamethyl-1-oxa-4,8-diaza-3-oxo-spiro-[4,5]-decan
61. 2-Ethyl-2-butyl-7,7,9,9-tetramethyl-1-oxa-4,8-diaza-3-oxo-spiro-[4,5]-decan
62. 2-Ethyl-2-pentyl-7,7,9,9-tetramethyl-1-oxa-4,8-diaza-3-oxo-spiro-[4,5]-decan
63. 2-Ethyl-2-iso-pentyl-7,7,9,9-tetramethyl-1-oxa-4,8-diaza-3-oxo-spiro-[4,5]-decan
64. 2,2,7,7,9,9-Hexamethyl-1-oxa-4,8-diaza-3-oxo-8-acetyl-spiro-[4,5]-decan
65. 2,2-Diethyl-7,7,9,9-tetramethyl-1-oxa-4,8-diaza-3-oxo-8-acetyl-spiro-[4,5]-decan
66. 2,2,4,4-Tetramethyl-7-oxa-3,14-diaza-13-oxo-dispiro-[5,1,4,2]-tetradecan
67. 2,2,4,4-Tetramethyl-7-oxa-3,15-diaza-14-oxo-dispiro-[5,1,5,2]-pentadecan
68. 2,2,4,4-Tetramethyl-7-oxa-3,21-diaza-20-oxo-dispiro-[5,1,11,2]-heneicosan

Geeignete Verbindungen der Formel (IVb) sind die Salze der Verbindungen der Formel (IVa) mit Protonsäuren, z.B. Chlorwasserstoff, Schwefelsäure, Phosphorsäure usw., beispielsweise die Hydrochloride der oben angegebenen Verbindungen Nr. 1 bis 68. Zur Erläuterung seien folgende Beispiele genannt:

69. 2,2,7,7,9,9-Hexamethyl-1-oxa-3,8-diaza-4-oxo-spiro-[4,5]-decan-hydrochlorid
70. 2,2,4,4-Tetramethyl-7-oxa-3,14-diaza-15-oxo-dispiro-[5,1,5,2]-pentadecan-hydrochlorid
71. 2,2,4,4-Tetramethyl-7-oxa-3,20-diaza-21-oxo-dispiro-[5,1,11,2]-heneicosan-hydrochlorid
72. 2,2,7,7,9,9-Hexamethyl-1-oxa-4,8-diaza-3-oxo-spiro-[4,5]-decan-hydrochlorid
73. 2,2-Diethyl-7,7,9,9-tetramethyl-1-oxa-4,8-diaza-3-oxo-spiro-[4,5]-decan-hydrochlorid
74. 2,2,4,4-Tetramethyl-7-oxa-3,14-diaza-13-oxo-dispiro-[5,1,4,2]-tetradecan-hydrochlorid
75. 2,2,4,4-Tetramethyl-7-oxa-3,15-diaza-14-oxo-dispiro-[5,1,5,2]-pentadecan-hydrochlorid
76. 2,2,4,4-Tetramethyl-7-oxa-3,21-diaza-20-oxo-dispiro-[5,1,11,2]-heneicosan-hydrochlorid

Beispiele für die Epoxide der Formel (V) sind:

77. 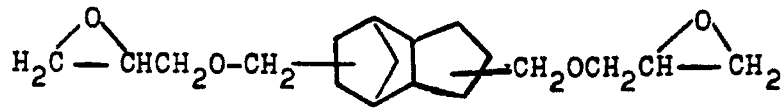

78. 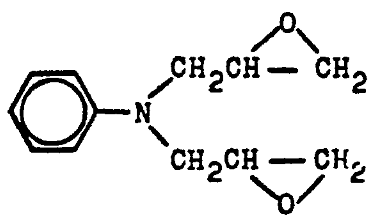

79. 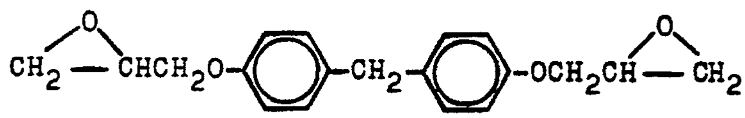

80. 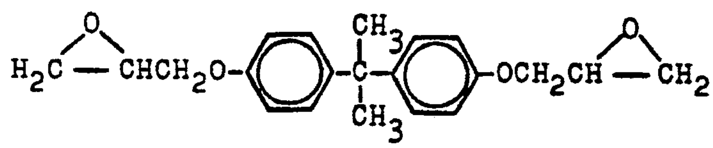

81. 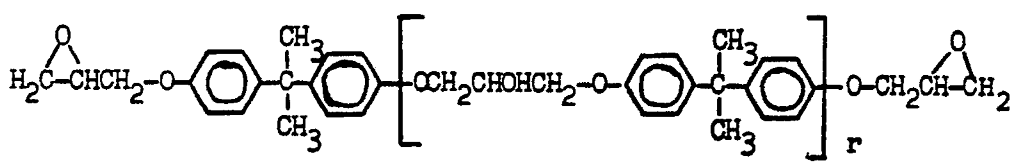

mit r = 1, 2, 3 ...

82. 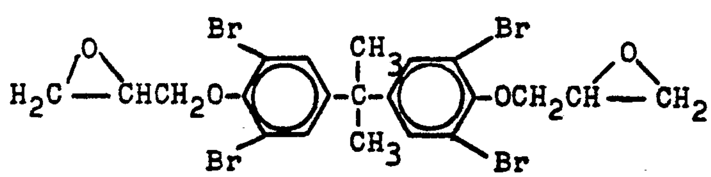

83. 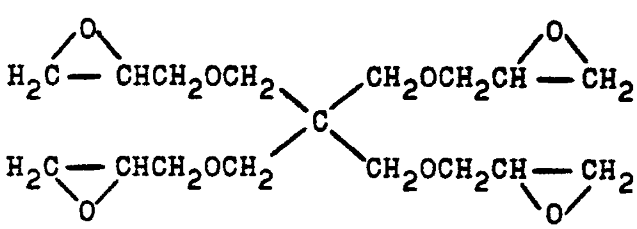

84. 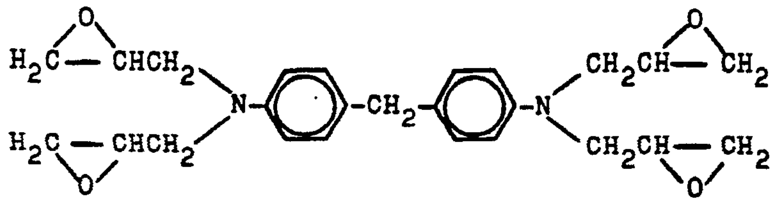

85. 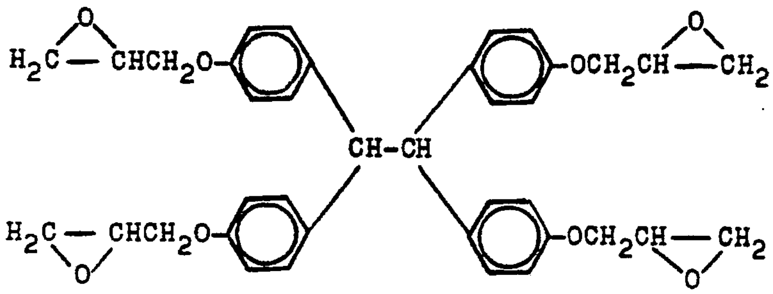

86. 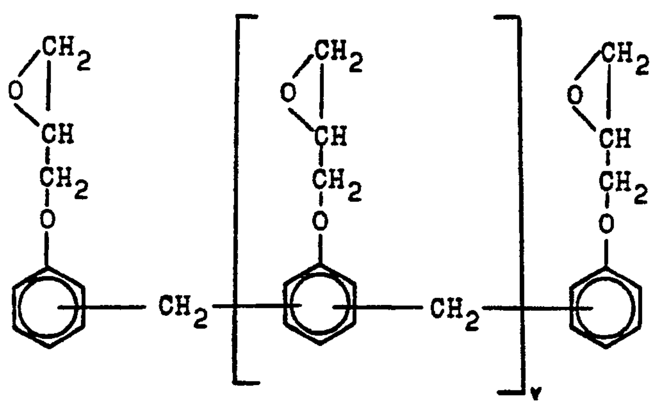

mit v = 1, 2, 3 ...

87. 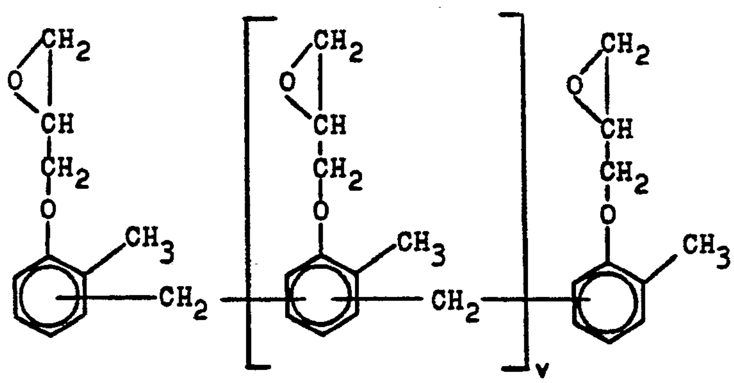

mit v = 1, 2, 3 ...

Die Verbindungen der Formel (V) müssen für die Umsetzung zu den erfindungsgemäßen Stabilisatoren nicht in reiner Form vorliegen, sondern können auch in Form der entsprechenden Epoxidharze technischer Qualität verwendet werden.

Die neuen Stabilisatoren lassen sich problemlos in die zu stabilisierenden Polymeren einarbeiten und sind hervorragend zum Stabilisieren derselben gegen den lichtinduzierten oxidativen Abbau, d.h. ihre

Schädigung durch Einwirkung von Sauerstoff, Wärme und Licht geeignet. Neben der ausgezeichneten Stabilisatorwirksamkeit zeichnet die neuen Stalilisatoren ihre sehr gute Verträglichkeit mit den zu stabilisierenden Polymeren aus.

Beispiele für erfolgreich zu stabilisierende Polymere sind: Polymere, die sich von einfach oder doppelt ungesättigten Kohlenwasserstoffen ableiten, z.B. Polyolefine wie Polyethylen, das gegebenenfalls vernetzt sein kann, Polypropylen, Polybuten-1, Polyisobuten, Polymethylbuten-1, Polymethylpenten-1, Polyisopren, Polybutadien, Polystyrol, Copolymere der den genannten Homopolymeren zugrundeliegenden Monomeren, wie Ethylen-Propylen-Copolymere, Propylen-Buten-1-Copolymere, Propylen-Isobuten-Copolymere, Styrol-Butadien-Copolymere sowie Terpolymere von Ethylen und Propylen mit einem Dien, wie z.B. Hexadien, Dicyclopentadien oder Ethylidennorbornen; Mischungen der obengenannten Homopolymeren, wie beispielsweise Gemische von Polypropylen und Polyethylen, Polypropylen und Polybuten-1, Polypropylen und Polyisobuten oder von Butadien-Acrylnitril-Copolymerisaten mit einem Styrol-Butadien-Copolymerisat.

Halogenhaltige Vinylpolymere, wie Polyvinylchlorid, Polyvinylidenchlorid, Polyvinylfluorid, Polychloropren, Chlorkautschuke sowie Copolymere von Vinylchlorid und Vinylidenchorid untereinander und mit anderer olefinisch ungesättigten Monomeren.

Polymere, die sich von a,ß-ungesättigten Säuren und deren Derivaten ableiten, wie Polyacrylate und Polymethacrylate, Polyacrylamide und Polyacrylnitril sowie deren Copolymere untereinander und mit anderen Vinylverbindungen, wie Acrylnitril-Butadien-Styrol-,Acrylnitril-Styrol- und Acrylnitril-Styrol-Acrylester-Copolymerisate.

Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin und deren Copolymere mit anderen Vinylverbindungen, wie Ethylen-Vinylacetat-Copolymere. Homo- und Copolymere, die sich von Epoxiden ableiten, wie Polyethylenoxid oder die Polymerisate, die sich von Bisglycidylethern ableiten.

Polyacetale, wie Polyoximethylen und Polyoxiethylen sowie solche Polyoximethylene, die als Comonomeres Ethylenoxid enthalten.

Polyurethane und Polyharnstoffe

Polycarbonat

Polyamide und Copolyamide, die sich von Diaminen und Dicarbonsäuren und/oder von Aminocarbonsäuren oder den entsprechenden Lactamen ableiten, wie Polyamid 6, Polyamid 6/6, Polyamid 6/10, Polyamid 11, Polyamid 12.

Polyester, die sich von Dicarbonsäuren und Dialkoholen und/oder von Hydroxicarbonsäuren oder den entsprechenden Lactonen ableiten, wie Polyethylenterephthalat, Polybutylenterephthalat, Poly-1,4-dimethylol-cyclohexanterephthalat.

Vernetzte Polymerisate, die sich von Aldehyden einerseits und Phenolen, Harnstoffen und Melaminen andererseits ableiten, wie Phenol-Formaldehyd-, Harnstoff-Formaldehyd- und Melamin-Formaldehydharze.

Die neuen Verbindungen lassen sich schließlich auch als Stabilisatoren auf dem Harz- und Lacksektor einsetzen. Beispiele sind duroplastische und thermoplastische Acrylharze, welche für Autolackierungen verwendet werden, Acrylharzlacke, das sind die üblichen Einbrennlacke, sowie ganz besonders Mischungen auf der Basis von heiß-vernetzbarem Acrylharz und Styrol sowie Lacke und Beschichtungen auf der Basis von Acryl-Melaminharz und Alkyd/Acryl/Melaminharz. Derartige Lacke können als weitere Zusatzstoffe andere übliche Lichtschutzmittel, phenolische Antioxidantien, Pigmente, Farbstoffe, Metalldesaktivatoren etc. enthalten.

Von besonderer Bedeutung ist die Stabilisierung von Polyolefinen, Styrolpolymerisaten, Polyamiden, Poly-(meth)acrylaten und von Polyurethanen, für die sich die Verbindungen bevorzugt eignen. Beispiele hierfür sind Polyethylen hoher und niedriger Dichte, Polypropylen, Ethylen-Propylen-Copolymerisate, Polystyrol, Styrol-Butadien-Acrylnitril-Terpolymerisate, Mischungen von Polyolefinen oder von Styrolpolymerisaten sowie Polyurethane auf Polyether- oder Polyesterbasis.

Die neuen Stabilisatoren werden nach allgemein üblichen Methoden in die Polymermasse eingearbeitet. Die Einarbeitung kann beispielsweise durch Einmischen der Verbindungen und gegebenenfalls weiterer Additive in die Schmelze nach den in der Technik üblichen Methoden, vor oder während der Formgebung, oder auch durch Aufbringen der gelösten oder dispergierten Verbindungen auf das Polymere direkt oder Einmischen in eine Lösung, Suspension oder Emulsion desselben, gegebenenfalls unter nachträglichem Verdunstenlassen des Lösungsmittels, erfolgen. Die Mengen liegen bei 0,01 bis 5, vorzugsweise bei 0,05 bis 2,5 und insbesondere bei 0,1 bis 1,0 Gew.-%, bezogen auf das zu stabilisierende Material. Die neuen Verbindungen können auch in Form eines Masterbatches, der diese Verbindungen beispielsweise in einer Konzentration von 1 bis 50, vorzugsweise 2,5 bis 20 Gew.-% enthält, den zu stabilisierenden Kunststoffen zugesetzt werden.

Die durch den Zusatz der erfindungsgemäßen Substanzen stabilisierten Kunststoffe können gegebenenfalls noch andere bekannte und übliche Zusätze, wie beispielsweise Antioxidantien auf Phenol- und Sulfidbasis, Metalldesaktivatoren und Lichtschutzmittel, Phosphitstabilisatoren, Metallverbindungen, Epoxistabilisatoren und mehrwertige Alkohole enthalten.

Beispiele für Antioxidantien sind sterisch gehinderte Phenole wie 2,6-Di-tert.butyl-4-methylphenol, 4,4'-Butyliden-bis-(2,6-di-tert.-butylphenol), 4,4'-Thio-bis-(2-tert. -butyl-5-methylphenol), 2,5-Di-tert.-butyl-4-hydroxianisol, 2,2-Bis-(3,5-di-tert.-butyl-2-hydroxibenzyl)-malonsäuredioctadecylester, 1,3,5-Tris-(3,5-di-tert.-butyl-4-hydroxibenzyl)-2,4,6-trimethylbenzol, 2,4,6-Tri-(3,5-di-tert.-butyl-4-hydroxibenzyl)-phenol, phenolische Triazinverbindungen wie 1,3,5-Tris-(3,5-di-tert.-butyl-4-hydroxibenzyl)-isocyanurat, Ester oder Amide der ß-(3,5-Di-tert.-butyl-4-hydroxiphenyl)-propionsäure mit z.B. Octadecanol, 1,6-Hexandiol, 2,2'-Thioethylenglykol, Pentaerythrit und Tris-hydroxiethyl-isocyanurat, Hexamethylendiamin, Ester der 3,3-Bis-(3-tert.-butyl-4-hydroxiphenyl)-butansäuren mit z.B. Ethylenglykol, Thiodipropionsäureester mit Fettalkoholen, Ca- oder Ni-Salze des 3,5-Di-tert.-butyl-4-hydroxibenzylphosphonsäureethylesters, Dioctadecylsulfid und -disulfid.

Beispiele für Metalldesaktivatoren sind N,N'-Diphenyloxalsäurediamid, N-Salizylal-N'-salizyloylhydrazin, N,N'-Bis-salizyloylhydrazin, N,N'-Bis-(3,5-di-tert.butyl-4-hydroxy-phenylpropionyl)-hydrazin, 3-Salizyloylamino-1,2,4-triazol, Bis-benzyliden-oxalsäuredihydrazid, Tris-(2-tert.-butyl-4-thio(2'-methyl-4'-hydroxy-5'-tert.butyl)-phenyl-5-methyl|-phenylphosphit, 2,2'-Oxamido-bis-(ethyl-3-(3,5-di-tert.-butyl-4-hydroxiphenyl)-propionat|.

Zu den UV-Absorbern und Lichtschutzmitteln gehören 2-(2'-Hydroxiphenyl)-benztriazole wie z.B. das 5-Chlor-3',5'-di-tert.-butyl- und 5-Chlor-3',5'-di-tert.-amyl-Derivat, 2-Hydroxybenzophenone wie z.B. das 4-Heptoxi-, das 4-Octoxi-oder 4-Decyloxi-Derivat, Salizylate wie Octylphenylsalizylat, Nickelkomplexe wie z.B. der Komplex mit 2,2'-Thio-bis-4-(1,1,3,3-tetramethylbutyl)-phenol und Butylamin oder anderen Aminen oder mit 2-Hydroxi-4-octoxi-benzophenon, Dialkyldithiocarbaminsäuren oder Dialkyldithiophosphonsäuren, Oxalsäurediamide und sterisch gehinderte Amine, Z.B. Bis(2,2,6,6-tetramethyl-4-piperidinyl)sebacat, Polyester der Bernsteinsäure mit N-(2-hydroxyethyl)-2,2,6,6-tetramethyl-4-hydroxi-piperidin, N,N'-Bis(2,2,6,6-tetramethyl-4-piperidinyl)-hexamethylendiamin, 2,2,4,4-Tetramethyl-7-oxa-3,20-diaza-21-oxo-dispiro-[5,1,11,2]-heneicosan, 1,1'-(1,2-ethandiyl)-bis-(3,3,5,5-tetramethylpiperazinon), Kondensationsprodukt von N,N'-Bis-(2,2,6,6-tetramethyl-4-piperidyl)hexamethylendiamin mit Dibromethan oder mit 4-tert.Octylamino-2,6-dichlor-1,3,5-triazin oder mit 4-(N-Morpholinyl)-2,6-dichlor-1,3,5-triazin, Tetrakis-(2,2,6,6-tetramethyl-4-piperidyl-1,2,3,4-butantetracarbonsäure.

Als Phosphite sind aliphatische, aromatische oder aliphatisch-aromatische wie z.B. Trisnonylphenylphosphit, Tris-(2,4-di-tert.-butylphenyl)-phosphit,Tris-(2-tert.-butylphenyl)-phosphit oder auch Ester des Pentaerythritphosphits zu nennen.

Als Stabilisatoren bekannte Metallverbindungen sind z.B.: Calcium-, Barium-, Strontium-, Zink-, Cadmium-, Magnesium-, Aluminium- und Bleiseifen aliphatischer Carbonsäuren oder Oxicarbonsäuren mit etwa 12 bis 32 C-Atomen, Salze der genannten Metalle mit aromatischen Carbonsäuren wie Benzoate oder Salizylate sowie (Alkyl-)-Phenolate dieser Metalle, ferner Organozinnverbindungen, wie z.B. Dialkylzinnthioglykolate und -carboxilate sowie Metalloxide, z.B. Oxide des Calcium, Magnesium, Zink, Aluminium oder des Silicium.

Bekannte Epoxistabilisatoren sind z.B. epoxidierte höhere Fettsäuren wie epoxidiertes Sojabohnenöl, Leinöl oder epoxidiertes Butyloleat, Epoxide langkettiger Olefine, sowie Polyetherepoxide.

Mehrwertige Alkohole können beispielsweise Pentaerythrit, Trimethylolpropan, Sorbit oder Mannit sein, d.h. bevorzugt Alkohole mit 5 oder 6 C-Atomen und 2 bis 6 OH-Gruppen.

Eine wirksame Stabilisatorkombination für Poly-$\alpha$-Olefine, wie z.B. Hoch-, Mittel- und Niederdruckpolymerisate von $C_2$- bis $C_4$-$\alpha$-Olefinen, insbesondere Polyethylen und Polypropylen oder von Copolymerisaten derartiger $\alpha$-Olefine besteht, bezogen auf 100 Gewichtsteile Polymer, beispielsweise aus 0,01 bis 5 Gewichtsteilen einer der erfindungsgemäß zu verwendenden Verbindungen, 0,05 bis 5 Gewichtsteilen eines phenolischen Stabilisators, gegebenenfalls 0,01 bis 5 Gewichtsteilen eines schwefelhaltigen Costabilisators sowie gegebenenfalls 0,01 bis 3 Gewichtsteilen einer basischen oder neutralen Metallseife, wie z.B. Calciumstearat oder Zinkstearat oder der entsprechenden Oxide, gegebenenfalls 0,01 bis 5 Gewichtsteile eines Phosphit- oder Phosphonitstabilisators sowie gegebenenfalls 0,01 bis 5 Gewichtsteilen eines bekannten UV-Stabilisators aus der Gruppe der Alkoxihydroxibenzophenone, 4-Hydroxiphenylbenzotriazole, Benzylidenmalonsäuremononitrilester oder der sog. Quencher, wie z.B. Nickelchelate. Als sonstige übliche Zusätze sind z.B. Weichmacher, Gleitmittel, Emulgatoren, Füllstoffe wie z.B. Kreide, Talkum, Asbest, Pigmente, optische Aufheller, Flammschutzmittel und Antistatika anzusehen.

Die erfindungsgemäß stabilisierten Kunststoffe können in verschiedener Form angewendet werden, z.B. als Folien, Fasern, Bändchen, Profile oder als Bindemittel für Lacke, Klebemittel oder Kitte.

Der weiteren Erläuterung der Erfindung dienen nachstehende Beispiele.

Beispiel 1

2,2-Bis-{[2-hydroxy-3-(2,2,4,4-tetramethyl-7-oxa-3,20-diaza-21-oxo-dispiro-[5.1.11.2]-heneicosyl-20)-1-propoxy]-phenyl-4}-propan

In einer 1 l-Rührapparatur mit Tropftrichter, Rückflußkühler, Innenthermometer und Rührer wurden 250 ml Toluol vorgelegt und 72,8 g 2,2,4,4-Tetramethyl-7-oxa-3,20-diaza-21-oxo-dispiro-[5.1.11.2]-heneicosan (Edukt 1, Verb. Nr. 30), 1 g Benzyltriethylammoniumchlorid, 38,5 g Bisphenol-A-diglycidylether (Edukt 2, Verb. Nr. 80) und 4 g 50%ige Natronlauge zugegeben. Nach dem Aufheizen des Ansatzes auf 90 °C wurden nochmals 12 g 50%ige Natronlauge zugetropft und 6 h weiter gerührt. Anschließend wurde 3mal mit 100 ml Wasser aufgeschüttelt. Die organische Phase wurde mit $Na_2SO_4$ getrocknet und das Toluol abdestilliert. Bei ca. 180 °C wurde der Rückstand 1/2 h im Vakuum getrocknet. Man erhielt 108,2 g farblose Produktschmelze, die nach dem Abkühlen gemahlen wurde. Das amorphe Produkt hatte einen Glaspunkt von 91 - 95 °C, einen Erweichungspunkt von 122 °C und einen Tropfpunkt von 134 °C. Die Molmasse wurde zu 1100 bestimmt (berechnet 1068).

Beispiel 2

2,2-Bis-{[2-hydroxy-3-(2,2,4,4-tetramethyl-7-oxa-3,20-diaza-21-oxo-dispiro-[5.1.11.2]-heneicosyl-20)-1-propoxy]-phenyl-4}-propan

Entsprechend der Verfahrensweise von Beispiel 1 wurden 80,1 g 2,2,4,4-Tetramethyl-7-oxa-3,20-diaza-21-oxo-dispiro-[5.1.11.2]-heneicosan-hydrochlorid umgesetzt, wobei die angegebene Natronlaugemenge auf 33 g 50%ige Natronlauge erhöht wurde. Ausbeute 107,9 g des gleichen Produktes wie in Beispiel 1.

Beispiel 3

2,2-Bis-{[2-hydroxy-3-(2,2,7,7,9,9-hexamethyl-1-oxa-4,8-diaza-3-oxo-spiro-[4,5]-decanyl-4)-1-propoxy]-phenyl-4}-propan

In einer 1 l-Rührapparatur mit Innenthermometer, Tropftrichter und Rückflußkühler wurden 250 ml Toluol vorgelegt, 55,3 g 2,2,7,7,9,9-Hexamethyl-1-oxa-4,8-diaza-3-oxo-spiro-[4,5]-decan-Hydrochlorid (Verb. Nr. 72), 2,2-Bis[(2,3-epoxipropyl)-phenyl-4-]propan (Verb. Nr. 80) und 19,8 g Kaliumhydroxid zugegeben und der Ansatz auf 90 °C aufgeheizt und 5 h bei dieser Temperatur gerührt. Anschließend wurde filtriert, das Toluol abdestilliert und der Rückstand bei 180 °C 1/2 h im Vakuum getrocknet. Man erhielt 83,5 g fast farblose Produktschmelze, die nach dem Abkühlen gemahlen wurde. Das Produkt hatte einen Erweichungspunkt von 100 °C und eine Molmasse von ca. 900.

Beispiele 4 bis 17

Gemäß Beispiel 1 bzw. 2 wurden folgende Produkte hergestellt:

| Beispiel Nr. | Edukt 1 Verb.-Nr. | (g) | Edukt 2 Verb.-Nr. | (g) | Produkt (g) | Ep (°C) |
|---|---|---|---|---|---|---|
| 4 | 30 | 72,8 | 79 | 35,0 | 100,2 | 116 |
| 5 | 30 | 72,8 | 77 | 42,0 | 107,6 | 99 |
| 6 | 30 | 72,8 | 84 | 28,0 | 102,6 | 154 |
| 7 | 30 | 72,8 | 86 | 38,0 | 95,5 | 148 |
| 8 | 29 | 56,0 | 80 | 38,5 | 80,3 | 115 |
| 9 | 29 | 56,0 | 79 | 35,0 | 65,0 | 118 |
| 10 | 70 | 31,7 | 80 | 19,3 | 46,3 | 115 |

| Beispiel Nr. | Edukt 1 Verb.-Nr. | (g) | Edukt 2 Verb.-Nr. | (g) | Produkt (g) | Ep (°C) |
|---|---|---|---|---|---|---|
| 11 | 43 | 24,0 | 80 | 19,3 | 40,2 | 90 |
| 12 | 43 | 24,0 | 77 | 21,0 | 40,0 | 68 |
| 13 | 43 | 24,0 | 86 | 18,0 | 38,5 | 106 |
| 14 | 43 | 24,0 | 83 | 17,0 | 36,1 | 86 |
| 15 | 43 | 24,0 | 84 | 22,0 | 35,5 | 152 |
| 16 | 43 | 24,0 | 85 | 19,2 | 38,2 | 80 |
| 17 | 72 | 27,7 | 80 | 19,3 | 41,7 | 90 |

Beispiel 18

Dieses Beispiel zeigt die Flüchtigkeit der Stabilisatoren gemäß Beispiel 1 und 10 im Vergleich zu Stabilisatoren der DE-PS 2 606 026 und DE-OS 31 04 294.

Die Flüchtigkeiten wurden in einer Apparatur zur thermogravimetrischen Analyse bestimmt. Gleiche Mengen (500 mg) der erfindungsgemäßen Verbindungen und der Vergleichssubstanzen wurden dazu im Stickstoffstrom (1 l/min) mit einer Aufheizgeschwindigkeit von 2 K/min bis auf 300°C erhitzt und der Substanzverlust in Gewichtsprozent gemessen.

| Stabilisator gem. Beispiel | Substanzverlust in Gew.-% beim Erreichen von ..°C 220 | 260 | 300 | 10 min bei 300 |
|---|---|---|---|---|
| 1 | 0 | 0,1 | 0,8 | 2,4 |
| 10 | 0 | 0,1 | 0,5 | 1,3 |
| Vergleich A[1)] | 0,4 | 0,7 | 2,5 | 6,0 |
| Vergleich B[2)] | 0,8 | 2,8 | 12,3 | 29,8 |

1) Stabilisator gemäß Beispiel 11 der DE-OS 31 04 294
2) Stabilisator gemäß Beispiel 31 der DE-PS 26 06 026

Beispiel 19

Eine Mischung bestehend aus

| | |
|---|---|
| 100 Gew.-T. | Polypropylenpulver (MFI 230/5; 4g/10 min, Dichte bei 23°C 0,903 g/cm³) |
| 0,1 Gew.-T. | Pentaerythrityl-tetrakis-2-(3,5-di-tert.-butyl-4-hydroxiphenyl)-propionat |
| 0,1 Gew.-T. | Calciumstearat und |
| 0,3 Gew.-T. | des zu prüfenden erfindungsgemäßen Stabilisators |

wurde in einem Laborschnellmischer homogenisiert. Diese Mischung wurde in einer Windsor-Spritzgußmaschine der Type SP 50 bei 240°C zu 60 x 60 x 1 mm-Platten verspritzt. Aus diesen Platten wurden T-förmige Prüfkörper nach DIN 53 383 ausgestanzt.

Zur Bestimmung der Wärmealterungsbeständigkeit wurden diese Prüfkörper in einem Umlufttrockenschrank in ein motorgetriebenes Gestell mit rotierenden Horden eingehängt und bei gleichmäßiger Frischluftzufuhr einer Temperaturbelastung von 140 °C unterworfen.

Die Zeit, nach der an einigen Stellen eine beginnende lokale Versprödung auftritt, nach DIN 53 383 gekennzeichnet durch Bildung verfärbter, trüber, teilweise abbröckelnder Stellen, wurde festgehalten.

Die Ergebnisse zeigt folgende Tabelle:

| Stabilisator gemäß Beispiel | beginnende Versprödung nach ... Tagen |
|---|---|
| 1 | 54 |
| 10 | 49 |
| Vergleich A | 41 |
| Vergleich B | 29 |
| Vergleich C[1)] | 29 |

[1)] ohne zu prüfenden Stabilisator

Beispiel 20

Die gemäß Beispiel 19 hergestellten Platten wurden in einem Schnellbelichtungs-Tischgerät Suntest der Fa. Original Hanau Quarzlampen GmbH ohne Zusatzfilter belichtet. Die Belichtungszeiten bis zu einer starken Rißbildung (visuelle Begutachtung mit einem Mikroskop) wurden wie folgt ermittelt:

| Stabilisator gemäß Beispiel | Belichtungszeit bis zum Auftreten starker Rißbildung |
|---|---|
| 1 | 505 |
| 10 | 505 |
| Vergleich A | 370 |
| Vergleich B | 473 |
| Vergleich C | 183 |

Beispiel 21 bis 24

Beispiel 21

Die im vorhergehenden Beispiel hergestellte stabilisierte Mischung wurde auf einer Labor-Folienblasanlage (Schneckendurchmeser 30 mm, Länge 20 D) zu Blasfolien von ca. 100 µm Dicke verarbeitet. Aus diesen Folien wurden Prüfkörper nach DIN 53 455, Form 3, verkleinert im Maßstab 1:3, ausgestanzt.

Zur Bestimmung der Lichtstabilität wurden diese Proben in einer Anordnung gemäß DIN 53 387 Nr. 5.1, Anmerkung 2, im Schnellbelichtungs- und Bewitterungsgerät (R)Xenotest 1200 (Original Hanau Quarzlampen GmbH) der Bestrahlung mit Wechsellicht unterworfen. Die Strahlungsintensität wurde durch UV-Filter (Spezialfilterglas d = 1,7 mm) moduliert. Die Lichtbeständigkeit wurde nach DIN 53 387 (17 min Trokkenperiode, 3 min Beregnen, Schwarztafeltemperatur 45 °C, relative Luftfeuchtigkeit wäkhrend der Trockenperiode 70 bis 75 %) geprüft. Gemessen wurde die Reißdehnung auf einer Zugprüfmaschine bei einer Abzugsgeschwindigkeit von 5 cm/min nach einer bestimmten Belichtungszeit.

Die Ergebnisse sind in folgender Tabelle zusammengestellt.

| Stabilisator gemäß Beispiel | Belichtungszeit bis zum 50 %-Wert (Reißdehnung bez. auf Ausgangswert) | 10 %-Wert |
|---|---|---|
| 1 | 300 h | 400 h |
| 10 | 360 h | 400 h |
| Vergleich A | 220 h | 240 h |
| Vergleich B | 280 h | 300 h |
| Vergleich C | 130 h | 140 h |

Beispiel 22

Wie in Beispiel 21 beschrieben wurden Folien und Prüfkörper aus folgender Mischung hergestellt:
100 Gew.-T. HD-Polyethylenpulver (MFI 190/5 1,7 g/10 min, Dichte 0,943 g/$cm^3$) und
0,15 Gew.-T. des zu prüfenden Stabilisators
Die Belichtung analog zu Beispiel 21 im Xenotest 1200 ergab folgende Ergebnisse:

| Stabilisator gemäß Beispiel | Restreißdehnung bez. auf den Ausgangswert nach 915 h Belichtung |
|---|---|
| 1 | 72 % |
| 10 | 83 % |
| Vergleich A | 35 % |
| Vergleich B | 53 % |

Beispiel 23

Die gleiche Messung wie in Beispiel 22 wurde mit folgender Mischung durchgeführt:
100 Gew.-T. HD-Polyethylenpulver gemäß Beispiel 22 und
0,3 Gew.-T. des zu prüfenden Stabilisators

| Stabilisator gemäß Beispiel | Restreißdehnung bez. auf den Ausgangswert nach 1194 h Belichtung |
|---|---|
| 1 | 96 % |
| 10 | 119 % |
| Vergleich A | 85 % |
| Vergleich B | 73 % |
| Vergleich C | 8 % |

Beispiel 24

Analog dem Beispiel 21 wurden aus folgender Mischung Prüfkörper hergestellt und belichtet:
100 Gew.-T. LD-Polyethylenpulver (MFI 190/2,16 2,5 g/10 min; Dichte 0,924 g/cm$^3$) und
0,3 Gew.-T. des zu prüfenden Stabilisators
Die Belichtungsergebnisse sind in der folgenden Tabelle zusammengestellt:

| Stabilisator gemäß Beispiel | Belichtungszeit bis zum 50 %-Wert (Restreißdehnung bez. auf den Ausgangswert) |
|---|---|
| 1 | 900 |
| Vergleich A | 700 |
| Vergleich B | 600 |
| Vergleich C | 600 |

## Patentansprüche

### Patentansprüche für folgende Vertragsstaaten : BE, CH, DE, FR, GB, IT, LI, NL, SE

**1.** Verbindungen der Formel (I)

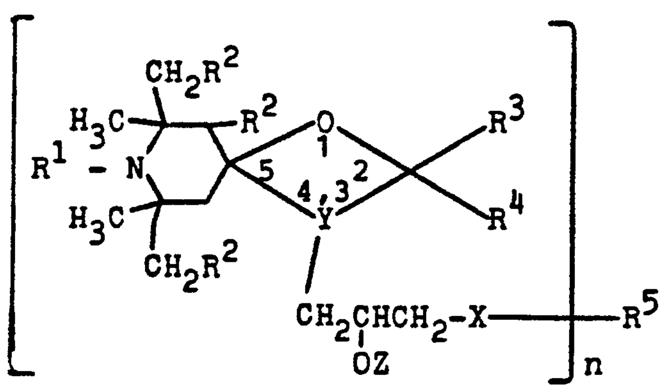

(I),

worin

| | |
|---|---|
| n | eine ganze Zahl von 1 bis 20 ist, |
| $R^1$ | Wasserstoff, $C_1$- bis $C_4$-Alkyl, Benzyl, Allyl, $C_2$- bis $C_{30}$-Alkanoyl, $C_2$- bis $C_{20}$-Alkenoyl, $C_7$- bis $C_{11}$-Aroyl, $C_8$- bis $C_{14}$-Arylalkanoyl oder $C_8$- bis $C_{20}$-Alkylaryl ist, |
| $R^2$ | Wasserstoff oder $C_1$- bis $C_4$-Alkyl ist, |
| $R^3$ und $R^4$ | gleich oder verschieden sind und Wasserstoff, $C_1$- bis $C_{18}$-Alkyl, unsubstituiertes oder durch Chlor oder $C_1$- bis $C_4$-Alkyl substituiertes Phenyl, unsubstituiertes oder durch $C_1$- bis $C_4$-Alkyl substituiertes $C_7$- bis $C_{14}$-Aralkyl oder zusammen mit dem diese Reste bindenden Kohlenstoffatom einen unsubstituierten oder durch bis zu vier $C_1$- bis $C_4$-Alkylgruppen substituierten $C_5$- bis $C_{12}$-Cycloalkyl-oder Piperidinring bedeuten, |
| $R^5$ bei n = 1 | einen mit 1 bis 4 $C_1$- bis $C_4$-Alkylgruppen substituierten Piperindinring bedeutet, |
| $R^5$ bei n = 2 | Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$- bis $C_4$-Alkyl-reste substituiert sein können, einen Rest |

$$-R^{6}-O-(CH_{2}\underset{OZ}{CH}CH_{2}-O-R^{6}-O|_{r} \quad \text{mit}$$

r = 1 bis 20 und $R^6$ gleich $C_2$- bis $C_{12}$-Alkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$- bis $C_4$-Alkyl-reste substituiert sein können, bedeutet,

$R^5$ bei n = 3 den Rest eines trifunktionellen Amins, den trifunktionellen Rest eines aliphatischen Alkohols, der noch weitere Hydroxylgruppen enthält, den trifunktionellen Rest eines Novolaks auf Basis Phenol, Kresol, Bisphenol-F oder Bisphenol-A bedeutet,

$R^5$ bei n = 4 den tetrafunktionellen Rest eines aliphatischen Alkohols oder Amins oder eines Novolaks auf Basis Phenol, Kresol, Bisphenol-F oder Bisphenol-A oder den Rest

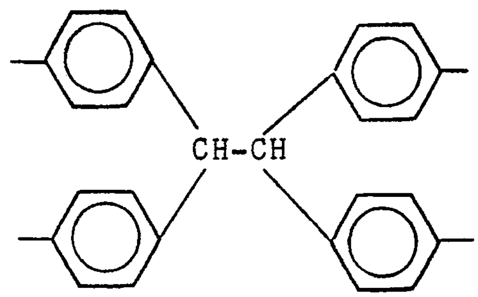

bedeutet,

$R^5$ bei n>4 einen polyfunktionellen Rest eines Polyols, eines Polyamins, eines Novolaks auf Basis Phenol, Kresol, Bisphenol-F oder Bisphenol-A bedeutet,

Y 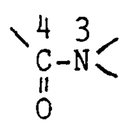 oder 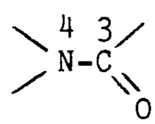 bedeutet und

die Ringpositionen 3, 4 in Formel (I) einnimmt,

X -O- oder $-N(R^7)-$ darstellt, wobei

$R^7$ Wasserstoff, $C_1$- bis $C_{30}$-Alkyl, ein durch 1 bis 4 $C_1$- bis $C_4$-Alkylgruppen substituierter Piperidinring oder ein Rest der Formel (II)

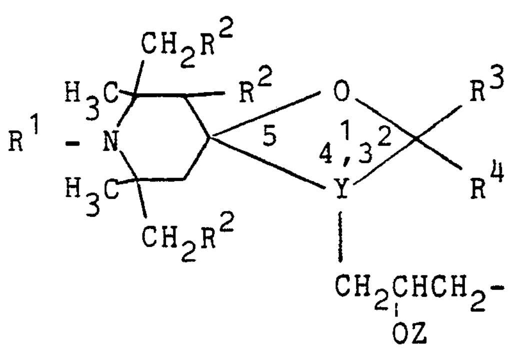

(II)

worin $R^1$, $R^2$, $R^3$, $R^4$ und Y die oben angegebenen Bedeutungen haben, ist,

Z Wasserstoff oder ein Rest der Formel (III)

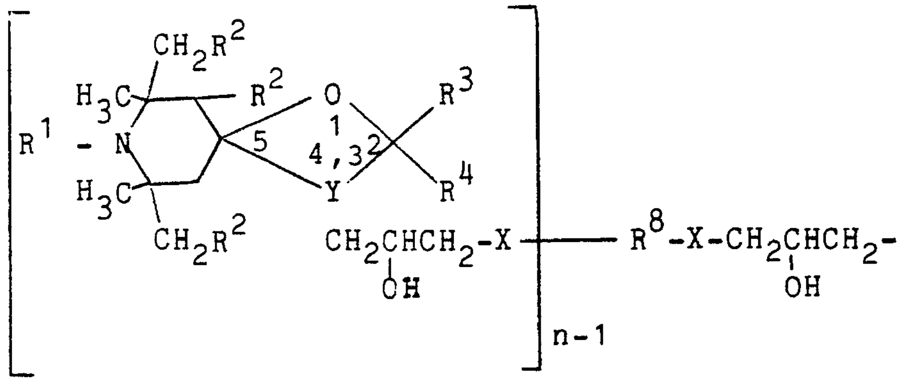

(III)

worin $R^1$, $R^2$, $R^3$, $R^4$, X, Y, Z und n die oben angegebenen Bedeutungen haben, oder ein Rest der Formel (II) ist, und

$R^8$ die Bedeutung von $R^5$ hat und zusätzlich noch

bei n = 1 Wasserstoff, $C_1$- bis $C_{30}$-Alkyl, Aryl, Aralkyl, Alkylaryl oder ein durch 1 bis 4 $C_1$- bis $C_4$-Alkylgruppen substituierter Piperidinring und

bei n = 2 $C_2$- bis $C_{18}$-Alkylen oder Phenylen ist, wobei dann X nur

$$-\underset{R^7}{\underset{|}{N}}-$$

sein darf, und

die Gruppierung

$$[-\underset{R^7}{\underset{|}{N}}-]_n R^5$$

aus Formel (I) zusätzlich

bei n = 2 für

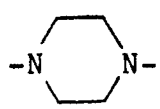 oder $R^7\underset{|}{N}-(CH_2)_s-\underset{|}{N}-R^7$ und

bei n > 2 für ein Polyamin der Formeln

$$R^7-\underset{|}{N}-(CH_2)_s-\underset{|}{N}-(CH_2)_t\underset{|}{N}-R^7$$

oder $R^7-\underset{|}{N}-(CH_2)_s\underset{|}{N}-(CH_2)_t-\underset{|}{N}-(CH_2)_s\underset{|}{N}-R^7$ steht,

wobei $R^7$ die oben angegebene Bedeutung hat und s und t ganze Zahlen von 2 bis 4 sind.

2. Verfahren zur Herstellung von Verbindungen der Formel I, dadurch gekennzeichnet, daß man ein Diazaoxaspirodecan der Formel (IVa) oder (IVb)

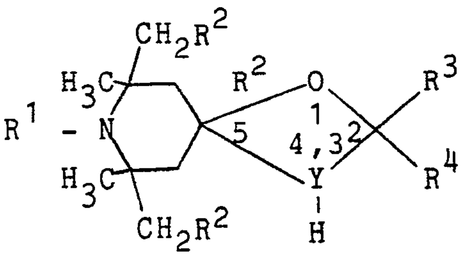

(IVa)

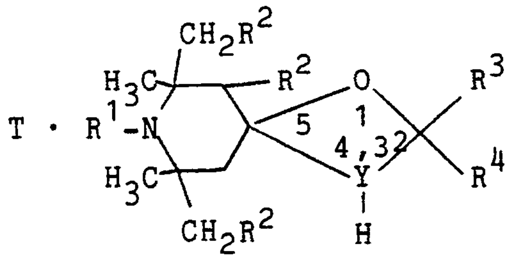

(IVb)

worin $R^1$, $R^2$, $R^3$, $R^4$ und Y die in Anspruch 1 angegebenen Bedeutungen haben und T eine nicht oxidierende Mineralsäure oder eine aliphatische oder aromatische Sulfon-oder Phosphonsäure, eine aliphatische Mono-, Di- oder Polycarbonsäure oder eine aromatische Mono- oder Dicarbonsäure ist,

A) mit einem Epoxid der Formel (Va) bzw. (Vb)

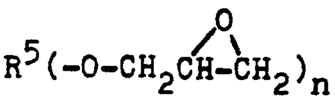

**(Va)**

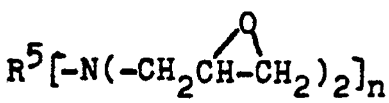

**(Vb)**

worin $R^5$ und n die in Anspruch 1 angegebenen Bedeutungen haben, in einem inerten Lösemittel in Gegenwart einer Base und gegebenenfalls eines Phasentransferkatalysators bei 40 bis 180 °C umsetzt, oder

B) mit Epichlorhydrin wie unter A) zu einer Verbindung der Formel (VI)

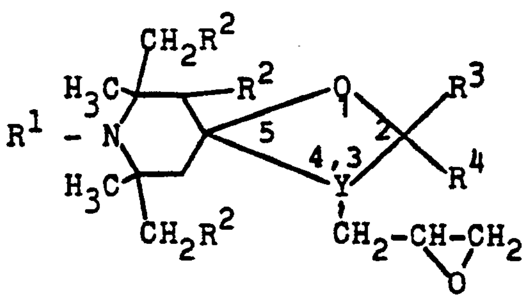

**(VI)**

und diese Verbindung dann mit einem Alkohol der Formel (VII)

$$R^5(-OH)_n \qquad \textbf{(VII)}$$

oder einem Amin der Formel (VIII)

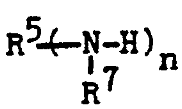

**(VIII)**

worin $R^1$, $R^2$, $R^3$, $R^4$, $R^5$, $R^7$, Y und n die im Anspruch 1 angegebenen Bedeutungen haben, oder einem Polyamin der Formel (IX)

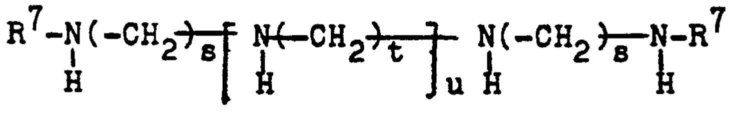 (IX)

worin $R^7$, s und t die oben angegebenen Bedeutungen haben und u eine ganze Zahl von 0 bis 3 ist, umsetzt.

3. Verwendung der Verbindungen nach Anspruch 1 zum Stabilisieren von synthetischen Polymeren.

4. Verwendung nach Anspruch 3, dadurch gekennzeichnet, daß das Polymere ein Polyolefin, ein Polyacrylat, ein Polymethacrylat oder ein Homo- oder Copolymerisat des Styrols ist.

5. Verwendung nach Anspruch 3, dadurch gekennzeichnet, daß das Polymere den Feststoffanteil eines Lackes darstellt.

6. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

7. Gegen UV-Zersetzung stabilisierte, synthetische Polymere, in denen 0,01 bis 5 Gewichtsteile, bezogen auf Polymeres, eines Stabilisators nach Anspruch 1 enthalten sind.

**Patentansprüche für folgenden Vertragsstaat : AT**

1. Verfahren zur Herstellung von Verbindungen der Formel (I)

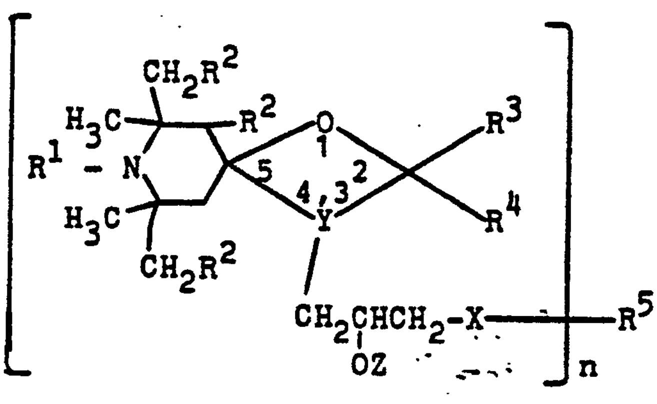 (I),

worin

| | |
|---|---|
| n | eine ganze Zahl von 1 bis 20 ist, |
| $R^1$ | Wasserstoff, $C_1$- bis $C_4$-Alkyl, Benzyl, Allyl, $C_2$- bis $C_{30}$-Alkanoyl, $C_2$- bis $C_{20}$-Alkenoyl, $C_7$- bis $C_{11}$-Aroyl, $C_8$- bis $C_{14}$-Arylalkanoyl oder $C_8$- bis $C_{20}$-Alkylaryl ist, |
| $R^2$ | Wasserstoff oder $C_1$- bis $C_4$-Alkyl ist, |
| $R^3$ und $R^4$ | gleich oder verschieden sind und Wasserstoff, $C_1$- bis $C_{18}$-Alkyl, unsubstituiertes oder durch Chlor oder $C_1$- bis $C_4$-Alkyl substituiertes Phenyl, unsubstituiertes oder durch $C_1$- bis $C_4$-Alkyl substituiertes $C_7$- bis $C_{14}$-Aralkyl oder zusammen mit dem diese Reste bindenden Kohlenstoffatom einen unsubstituierten oder durch bis zu vier $C_1$- bis $C_4$-Alkylgruppen substituierten $C_5$- bis $C_{12}$-Cycloalkyl-oder Piperidinring bedeuten, |
| $R^5$ bei n = 1 | einen mit 1 bis 4 $C_1$- bis $C_4$-Alkylgruppen substituierten Piperindinring bedeutet, |
| $R^5$ bei n = 2 | Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese |

Reste auch durch Brom oder Chlor bzw. $C_1$- bis $C_4$-Alkyl-reste substituiert sein können, einen Rest

$$-R^6-O-(CH_2\underset{OZ}{C}HCH_2-O-R^6-O]_r \quad \text{mit}$$

r = 1 bis 20 und $R^6$ gleich $C_2$- bis $C_{12}$-Alkylen, Cycloalkylen, Dicycloalkylen, Tricycloalkylen, Bismethylenmonocycloalkylen, Bismethylendicycloalkylen, Bismethylentricycloalkylen, Arylen, Bisarylenalkyl, wobei diese Reste auch durch Brom oder Chlor bzw. $C_1$- bis $C_4$-Alkyl-reste substituiert sein können, bedeutet,

$R^5$ bei n = 3 den Rest eines trifunktionellen Amins, den trifunktionellen Rest eines aliphatischen Alkohols, der noch weitere Hydroxylgruppen enthält, den trifunktionellen Rest eines Novolaks auf Basis Phenol, Kresol, Bisphenol-F oder Bisphenol-A bedeutet,

$R^5$ bei n = 4 den tetrafunktionellen Rest eines aliphatischen Alkohols oder Amins oder eines Novolaks auf Basis Phenol, Kresol, Bisphenol-F oder Bisphenol-A oder den Rest

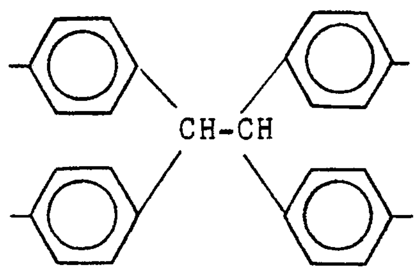

bedeutet,

$R^5$ bei n>4 einen polyfunktionellen Rest eines Polyols, eines Polyamins, eines Novolaks auf Basis Phenol, Kresol, Bisphenol-F oder Bisphenol-A bedeutet,

Y 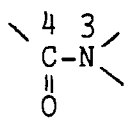 oder 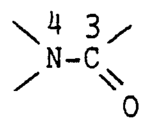 bedeutet und

die Ringpositionen 3, 4 in Formel (I) einnimmt,

X $-O-$ oder $-\underset{R^7}{N}-$ darstellt, wobei

$R^7$ Wasserstoff, $C_1$- bis $C_{30}$-Alkyl, ein durch 1 bis 4 $C_1$- bis $C_4$-Alkylgruppen substituierter Piperidinring oder ein Rest der Formel (II)

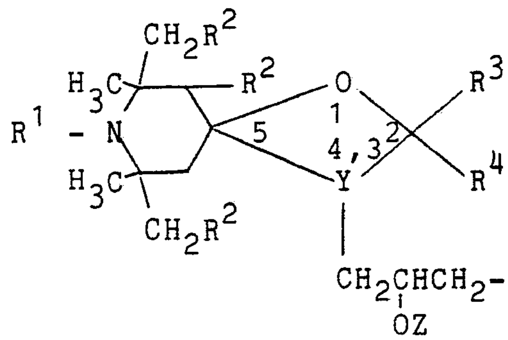

(II)

worin $R^1$, $R^2$, $R^3$, $R^4$ und Y die oben angegebenen Bedeutungen haben, ist,

Z Wasserstoff oder ein Rest der Formel (III)

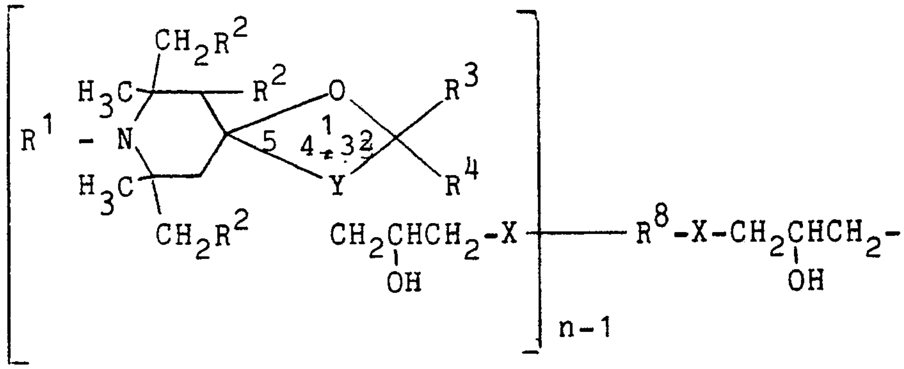

(III)

worin $R^1$, $R^2$, $R^3$, $R^4$, X, Y, Z und n die oben angegebenen Bedeutungen haben, oder ein Rest der Formel (II) ist, und

$R^8$ die Bedeutung von $R^5$ hat und zusätzlich noch

bei n = 1 Wasserstoff, $C_1$- bis $C_{30}$-Alkyl, Aryl, Aralkyl, Alkylaryl oder ein durch 1 bis 4 $C_1$- bis $C_4$-Alkylgruppen substituierter Piperidinring und

bei n = 2 $C_2$- bis $C_{18}$-Alkylen oder Phenylen ist, wobei dann X nur

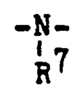

sein darf, und

die Gruppierung

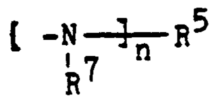

aus Formel (I) zusätzlich

bei n = 2 für

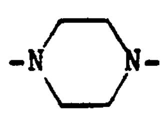 oder $R^7N-(CH_2)_s-N-R^7$ und

bei n > 2 für ein Polyamin der Formeln

$$R^7-\underset{|}{N}-(CH_2)_s-\underset{|}{N}-(CH_2)_t\underset{|}{N}-R^7$$

$$\text{oder}\quad R^7-\underset{|}{N}-(CH_2)_s\underset{|}{N}-(CH_2)_t-\underset{|}{N}-(CH_2)_s N-R^7 \quad \text{steht,}$$

wobei $R^7$ die oben angegebene Bedeutung hat und s und t ganze Zahlen von 2 bis 4 sind,

dadurch gekennzeichnet, daß man
ein Diazaspirodecan der Formel (IVa) oder (IVb)

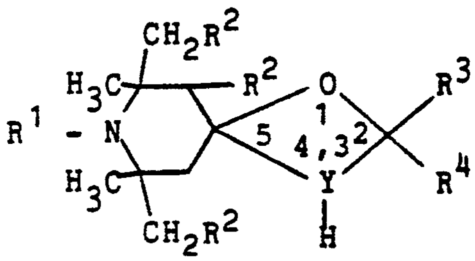
(IVa)

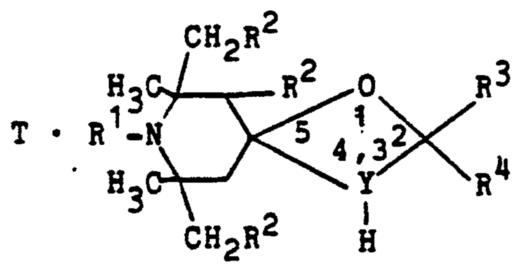
(IVb)

worin $R^1$, $R^2$, $R^3$, $R^4$ und Y die in Anspruch 1 angegebenen Bedeutungen haben und T eine nicht oxidierende Mineralsäure oder eine aliphatische oder aromatische Sulfonoder Phosphonsäure, eine aliphatische Mono-, Di- oder Polycarbonsäure oder eine aromatische Mono- oder Dicarbonsäure ist,

A) mit einem Epoxid der Formel (Va) bzw. (Vb)

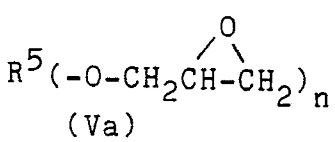
(Va)

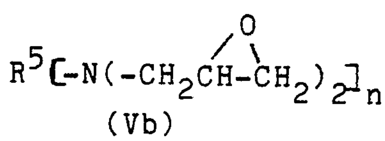
(Vb)

worin $R^5$ und n die oben angegebenen Bedeutungen haben, in einem inerten Lösemittel in Gegenwart einer Base und gegebenenfalls eines Phasentransferkatalysators bei 40 bis 180 °C umsetzt, oder

B) mit Epichlorhydrin wie unter A) zu einer Verbindung der Formel (VI)

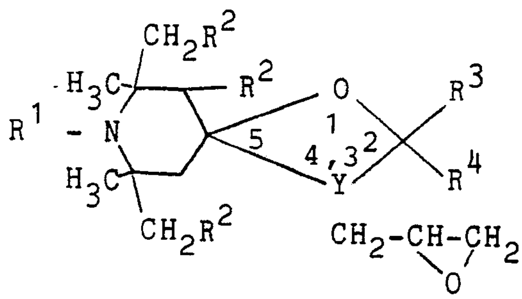
(VI)

und diese Verbindung dann mit einem Alkohol der Formel (VII)

$$R^5(-OH)_n \qquad \text{(VII)}$$

oder einem Amin der Formel (VIII)

$$R^5(-\underset{\substack{| \\ R^7}}{N}-H)_n \qquad \text{(VIII)}$$

worin $R^1$, $R^2$, $R^3$, $R^4$, $R^5$, $R^7$, Y und n die oben angegebenen Bedeutungen haben, oder einem Polyamin der Formel (IX)

$$R^7-\underset{\substack{| \\ H}}{N}(-CH_2)_s\left[\underset{\substack{| \\ H}}{N}(-CH_2)_t\right]_u\underset{\substack{| \\ H}}{N}(-CH_2)_s\underset{\substack{| \\ H}}{N}-R^7 \qquad \text{(IX)}$$

worin $R^7$, s und t die oben angegebenen Bedeutungen haben und u eine ganze Zahl von 0 bis 3 ist, umsetzt.

**2.** Verwendung der Verbindungen nach Anspruch 1 zum Stabilisieren von synthetischen Polymeren.

**3.** Verwendung nach Anspruch 3, dadurch gekennzeichnet, daß das Polymere ein Polyolefin, ein Polyacrylat, ein Polymethacrylat oder ein Homo- oder Copolymerisat des Styrols ist.

**4.** Verwendung nach Anspruch 3, dadurch gekennzeichnet, daß das Polymere den Feststoffanteil eines Lackes darstellt.

**5.** Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 5 Gewichtsteile, bezogen auf Polymeres, eines Stabilisators nach Anspruch 1 zusetzt.

**6.** Gegen UV-Zersetzung stabilisierte, synthetische Polymere, in denen 0,01 bis 5 Gewichtsteile, bezogen auf Polymeres, eines Stabilisators nach Anspruch 1 enthalten sind.

**Claims**

**Claims for the following Contracting States : BE, CH, DE, FR, GB, IT, LI, NL, SE**

**1.** A compound of the formula (I)

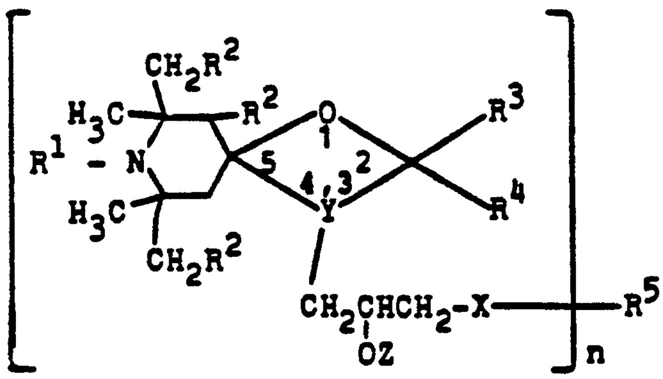

(I)

in which

| | |
|---|---|
| n | is a whole number from 1 to 20, |
| $R^1$ | is hydrogen, $C_1$- to $C_4$-alkyl, benzyl, allyl, $C_2$-to $C_{30}$-alkanoyl, $C_2$- to $C_{20}$-alkenoyl, $C_7$- to $C_{11}$-aroyl, $C_8$- to $C_{14}$-arylalkanoyl or $C_8$- to $C_{20}$-alkylaryl, |
| $R^2$ | is hydrogen or $C_1$- to $C_4$-alkyl, |
| $R^3$ and $R^4$ | are identical or different and denote hydrogen, $C_1$- to $C_{18}$-alkyl, phenyl which is unsubstituted or substituted by chlorine or $C_1$- to $C_4$-alryl, $C_7$- to $C_{14}$-aralkyl which is unsubstituted or substituted by $C_1$- to $C_4$-alkyl, or together with the carbon atom connecting these radicals a $C_5$- to $C_{12}$-cycloalkyl or piperidine ring which is unsubstituted or substituted by up to four $C_1$- to $C_4$-alkyl groups, |
| $R^5$ when n = 1 | denotes a piperidine ring which is substituted by 1 to 4 $C_1$- to $C_4$-alkyl groups, |
| $R^5$ when n = 2 | denotes cycloalkylene, dicycloalkylene, tricycloalkylene, bismethylenemonocycloalkylene, bismethylenedicycloalkylene, bismethylenetricycloalkylene, arylene, bisarylenealkyl, which radicals can also be bromine- or chlorine- or $C_1$- to $C_4$-alkyl-substituted, a radical |

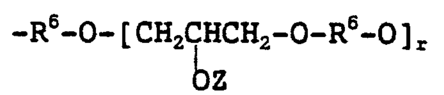 with

r = 1 to 20 and $R^6$ = $C_2$- to $C_{12}$-alkylene, cycloalkylene, dicycloalkylene, tricycloalkylene, bismethylenemonocycloalkylene, bismethylenedicycloallene, bismethylenetricycloalkylene, arylene, bisarylenealkyl, which radicals can also be bromine-or chlorine- or $C_1$ to $C_4$-alkyl-substituted,

| | |
|---|---|
| $R^5$ when n = 3 | denotes the radical of a trifunctional amine, the trifunctional radical of an aliphatic alcohol which contains further hydroxyl groups, the trifunctional radical of a novolak based on phenol, cresol, bisphenol-F or bisphenol-A, |
| $R^5$ when n = 4 | denotes the tetrafunctional radical of an aliphatic alcohol or amine or of a novolak based on phenol, cresol, bisphenol-F or bisphenol-A or the radical |

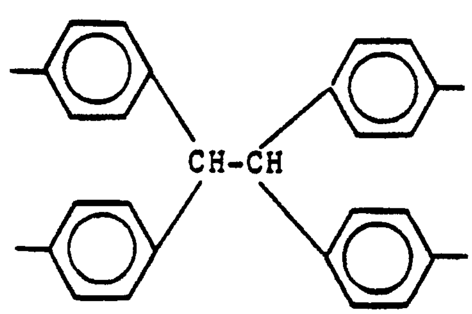

| | |
|---|---|
| $R^5$ when n > 4 | denotes a polyfunctional radical of a polyol, of a polyrnine, of a novolak based on |

phenol, cresol, bisphenol-F or bisphenol-A,

Y denotes

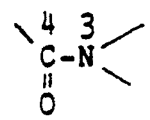 or 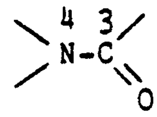

and occupies ring positions 3, 4 in the formula (I),

X represents -O- or

$$-\underset{R^7}{N}-,$$

where $R^7$ is hydrogen, $C_1$- to $C_{30}$-alkyl, a piperidine ring which is substituted by 1 to 4 $C_1$- to $C_4$-alkyl groups, or a radical of the formula (II)

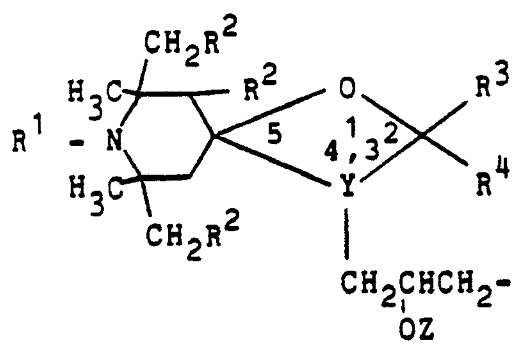 (II)

in which R', $R^2$, $R^3$, $R^4$ and Y have the abovementioned meanings,

Z is hydrogen or a radical of the formula (III)

Z

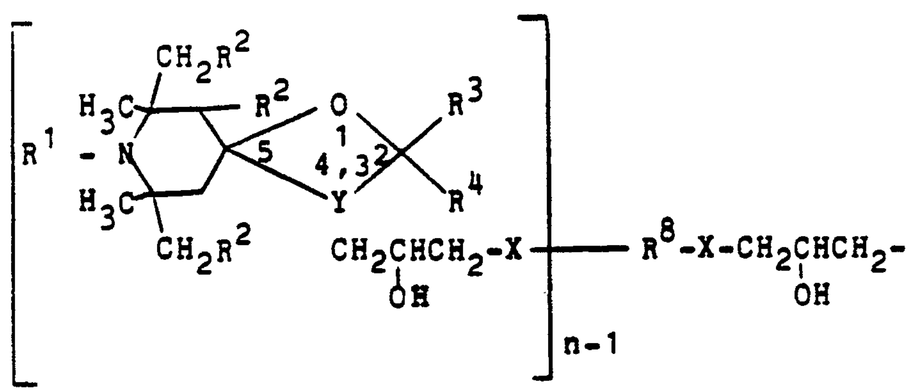 (III)

in which R', $R^2$, $R^3$, $R^4$, X, Y, Z and n have the abovementioned meanings, or a radical of the formula (II), and

$R^8$ has the meaning of $R^5$ and additionally stands

when n = 1 for hydrogen, $C_1$,- to $C_{30}$-alkyl, aryl, aralkyl, alkylaryl or a piperidine ring which is substituted by 1 to 4 $C_1$- to $C_4$-alkyl groups

and

when n = 2 $C_2$- to $C_{18}$-alkylene or phenylene, in which case X may then only be

$$-\underset{R^7}{\underset{|}{N}}-,$$

and the grouping

$$[-\underset{R^7}{\underset{|}{N}}-]_n R^5$$

from the formula (I) additionally stands

when n = 2 for

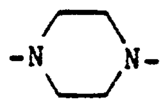

or $R^7\underset{|}{N}-(CH_2)_s-\underset{|}{N}-R^7$

and

when n > 2 for a polyamine of the formulae

$$R^7-\underset{|}{N}-(CH_2)_s-\underset{|}{N}-(CH_2)_t\underset{|}{N}-R^7$$

or

$$R^7-\underset{|}{N}-(CH_2)_s\underset{|}{N}-(CH_2)_t-\underset{|}{N}-(CH_2)_s\underset{|}{N}-R^7$$

where $R^7$ has the abovementioned meaning and s and t are whole numbers from 2 to 4.

2. A process for preparing a compound of the formula I, which comprises reacting a diazaoxaspirodecane of the formula (IVa) or (IVb)

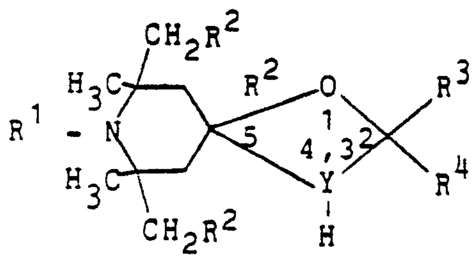

(IVa)

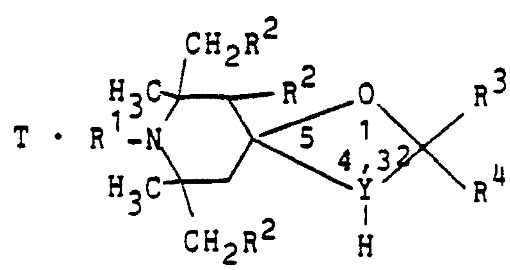

(IVb)

in which $R^1$, $R^2$, $R^3$, $R^4$ and Y have the meanings specified in claim 1 and T is a nonoxidizing mineral acid or an aliphatic or aromatic sulfonic or phosphonic acid, an aliphatic mono-, di- or poly-carboxylic acid or an aromatic mono- or di-carboxylic acid,

A) with an epoxy of the formula (Va) or (Vb)

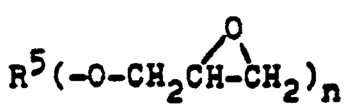

(Va)

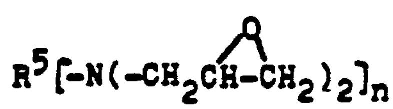

(Vb)

in which $R^5$ and n have the meanings specified in claim 1, in an inert solvent in the presence of a base and if desired of a phase transfer catalyst at 40 to 180 °C, or
B) with epichlorohydrin as under A) to give a compound of the formula (VI)

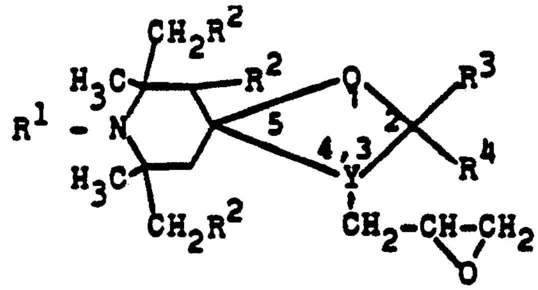

(VI)

and then reacting this compound with an alcohol of the formula (VII)

$R^5(OH)_n$ (VII)

or an amine of the formula (VIII)

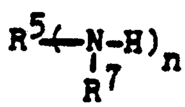

(VIII)

in which $R^1$, $R^2$, $R^3$, $R^4$, $R^5$, $R^6$, $R^7$, Y and n have the meanings specified in claim 1, or a polyamine of the formula (IX)

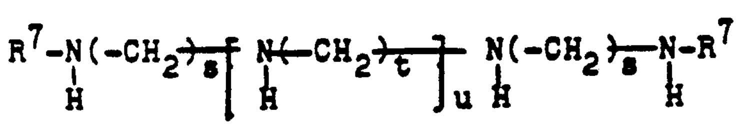

(IX)

in which $R^7$, s and t have the abovementioned meanings and u is a whole number from 0 to 3.

**3.** Use of the compound as claimed in claim 1, for stabilizing synthetic polymers.

**4.** Use as claimed in claim 3, wherein the polymer is a polyolefin, a polyacrylate, a polymethacrylate or a homopolymer or copolymer of styrene.

**5.** Use as claimed in claim 3, wherein the polymer is the solids content of a paint.

**6.** A process for stabilizing synthetic polymers against the damaging influence of light, which comprises adding to the polymers, if desired in addition to hitherto disclosed stabilizing substances, 0.01 to 5 parts by weight, based on a polymer, of a stabilizer as claimed in claim 1.

**7.** A synthetic polymer stabilized against UV decomposition and containing 0.01 to 5 parts by weight, based on a polymer, of a stabilizer as claimed in claim 1.

**Claims for the following Contracting State : AT**

**1.** A process for preparing a compound of the formula (I)

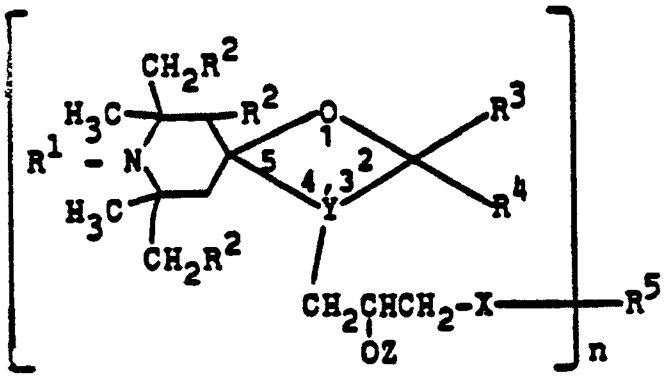

(I)

in which

n is a whole number from 1 to 20,

$R^1$ is hydrogen, $C_1$- to $C_4$-alkyl, benzyl, allyl, $C_2$- to $C_{30}$-alkanoyl, $C_2$- to $C_{20}$-alkenoyl, $C_7$- to $C_{11}$-aroyl, $C_8$- to $C_{14}$-arylalkanoyl or $C_8$- to $C_{20}$-alkylaryl,

$R^2$ is hydrogen or $C_1$- to $C_4$-alkyl,

$R^3$ and $R^4$ are identical or different and denote hydrogen, $C_1$- to $C_{18}$-alkyl, phenyl which is unsubstituted or substituted by chlorine or $C_1$- to $C_4$-alkyl, $C_7$- to $C_{14}$-aralkyl which is unsubstituted or substituted by $C_1$- to $C_4$-alkyl, or together with the carbon atom connecting these radicals a $C_5$- to $C_{12}$-cycloalkyl or piperidine ring which is unsubstituted or substituted by up to four $C_1$- to $C_4$-alkyl groups,

$R^5$ when n = 1 denotes a piperidine ring which is substituted by 1 to 4 $C_1$- to $C_4$-alkyl groups,

$R^5$ when n = 2 denotes cycloalkylene, dicycloalkylene, tricycloalkylene, bismethylenemonocycloalkylene, bismethylenedicycloalkylene, bismethylenetricycloalkylene, arylene, bisarylenealkyl, which radicals can also be bromine- or chlorine- or $C_1$- to $C_4$-alkyl-substituted, a radical

$-R^6-O-[CH_2CHCH_2-O-R^6-O]_r$ with
OZ

r = 1 to 20 and $R^6$ = $C_2$- to $C_{12}$-allene, cycloalkylene, dicycloalkylene, tricycloalkylene, bismethylenemonocycloalkylene, bismethylenedicycloalkylene, bismethylenetricycloalXylene, arylene, bisarylenealkyl, which radicals can also be bromineor chlorine- or $C_1$,- to $C_4$-alkyl-substituted,

$R^5$ when n = 3 denotes the radical of a trifunctional amine, the trifunctional radical of an aliphatic alcohol which contains further hydroryl groups, the trifunctional radical of a novolak based on phenol, cresol, bisphenol-F or bisphenol-A,

$R^5$ when n = 4 denotes the tetrafunctional radical of an aliphatic alcohol or amine or of a novolak based on phenol, cresol, bisphenol-F or bisphenol-A or the radical

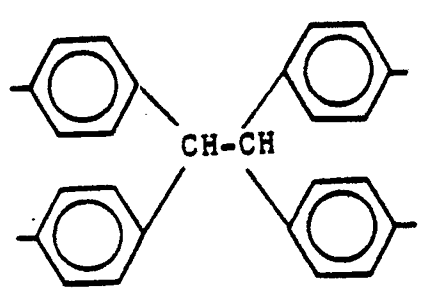

$R^5$ when n > 4 denotes a polyfunctional radical of a polyol, of a polyamine, of a novolak based on phenol, cresol, bisphenol-F or bisphenol-A,

Y denotes

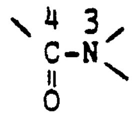 or 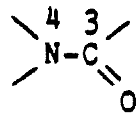

and occupies ring positions 3, 4 in the formula (I),

X represents -O- or

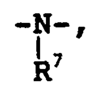

where $R^7$ is hydrogen, $C_1$- to $C_{30}$-alkyl, a piperidine ring which is substituted by 1 to 4 $C_1$,- to $C_4$-alkyl groups, or a radical of the formula (II)

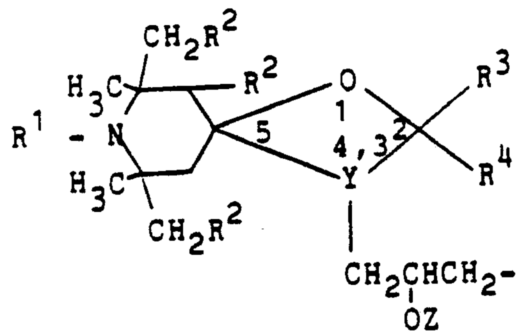

(II)

in which $R^1$, $R^2$, $R^3$, $R^4$ and Y have the abovementioned meanings,

Z is hydrogen or a radical of the formula (III)

Z

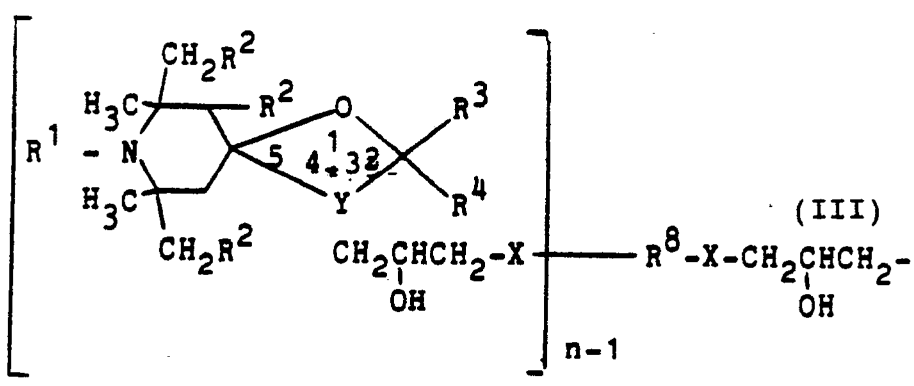

(III)

in which $R^1$, $R^2$, $R^3$, $R^4$, X, Y, Z and n have the abovementioned meanings, or a radical of the formula (II), and

$R^8$ has the meaning of $R^5$ and additionally stands

when n = 1 for hydrogen, $C_1$- to $C_{30}$-alkyl, aryl, aralkyl, alkylaryl or a piperidine ring which is substituted by 1 to 4 $C_1$- to $C_4$-alkyl groups
and

when n = 2 $C_2$- to $C_{18}$-alkylene or phenylene, in which case X may then only be

$$-\underset{\underset{R^7}{|}}{N}-,$$

and the grouping

$$[-\underset{\underset{R^7}{|}}{N}-]_n-R^5$$

from the formula (I) additionally stands

when n = 2 for

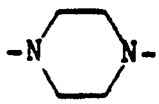 or $R^7\underset{|}{N}-(CH_2)_s-\underset{|}{N}-R^7$

and

when n > 2 for a polyamine of the formulae or

$$R^7-\underset{|}{N}-(CH_2)_s-\underset{|}{N}-(CH_2)_t\underset{|}{N}-R^7$$

$$R^7-\underset{|}{N}-(CH_2)_s\underset{|}{N}-(CH_2)_t-\underset{|}{N}-(CH_2)_s N-R^7$$

where $R^7$ has the abovementioned meaning and s and t are whole numbers from 2 to 4,

which comprises reacting a diazaoxaspirodecane of the formula (IVa) or (IVb)

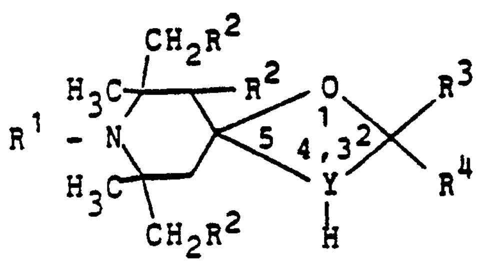

(IVa)

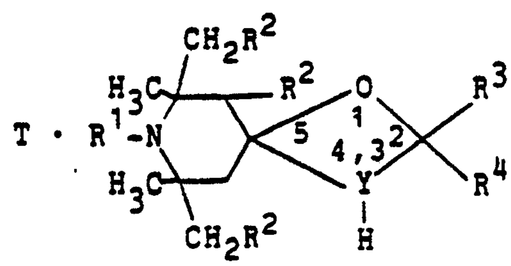

(IVb)

in which $R^1$, $R^2$, $R^3$, $R^4$ and Y have the meanings specified in claim 1 and T is a nonoxidizing mineral acid or an aliphatic or aromatic sulfonic or phosphonic acid, an aliphatic mono-, di- or poly-carborylic acid or an aromatic mono- or di-carborylic acid,

A) with an epoxy of the formula (Va) or (Vb)

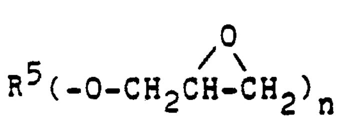

(Va)

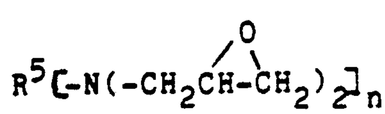

(Vb)

in which $R^5$ and n have the meanings specified above, in an inert solvent in the presence of a base and if desired of a phase transfer catalyst at 40 to 180° C, or
B) with epichlorohydrin as under A) to give a compound of the formula (VI)

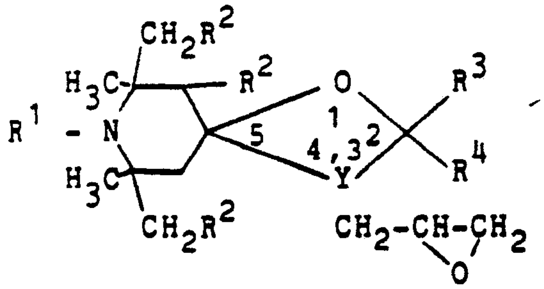

(VI)

and then reacting this compound with an alcohol of the formula (VII)

$R^5(-OH)_n$ (VII)

or an amine of the formula (VIII)

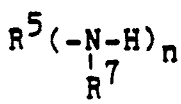

(VIII)

in which $R^1$, $R^2$, $R^3$, $R^4$, $R^5$, $R^7$, Y and n have the meanings specified above, or a polyamine of the formula (IX)

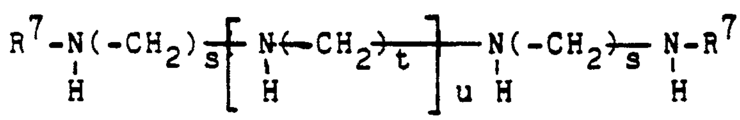

(IX)

in which $R^7$, s and t have the abovementioned meanings and u is a whole number from 0 to 3.

2. Use of the compound as claimed in claim 1, for stabilizing synthetic polymers.

3. Use as claimed in claim 3, wherein the polymer is a polyolefin, a polyacrylate, a polymethacrylate or a homopolymer or copolymer of styrene.

4. Use as claimed in claim 3, wherein the polymer is the solids content of a paint.

5. A process for stabilizing synthetic polymers against the damaging influence of light, which comprises adding to the polymers, if desired in addition to hitherto disclosed stabilizing substances, 0.01 to 5 parts by weight, based on a polymer, of a stabilizer as claimed in claim 1.

6. A synthetic polymer stabilized against UV decomposition and containing 0.01 to 5 parts by weight, based on a polymer, of a stabilizer as claimed in claim 1.

**Revendications**
**Revendications pour les Etats Contractants suivants : BE, CH, DE, PR, GB, IT, LU, NL, SE**

1. Composés de formule (I)

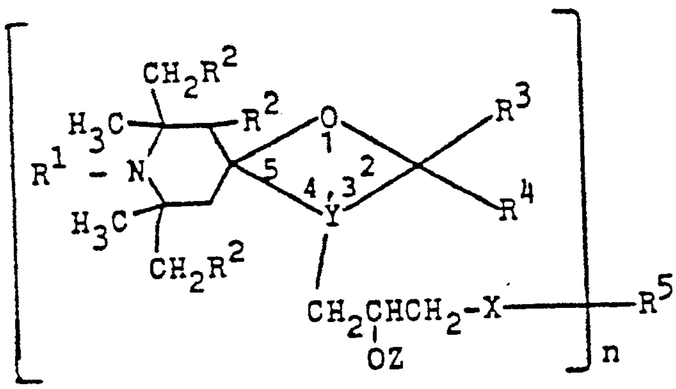

(I),

dans laquelle

n est un nombre entier valant 1 à 20 ;

$R^1$ représente un atome d'hydrogène ; un reste alkyle en $C_1$ à $C_4$, benzyle, allyle, alcanoyle en $C_2$ à $C_{30}$, alcénoyle en $C_2$ à $C_{20}$, aroyle en $C_7$ à $C_{11}$, arylalcanoyle en $C_8$ à $C_{14}$ ou alkylaryle en $C_8$ à $C_{20}$,

$R^2$ représente un atome d'hydrogène ou un reste alkyle en $C_1$ à $C_4$,

$R^3$ et $R^4$ sont identiques ou différents et représentent chacun un atome d'hydrogène, un reste alkyle en $C_1$ à $C_{18}$, un reste phényle (non substitué ou substitué par du chlore ou par un groupe alkyle en $C_1$ à $C_4$), un reste aralkyle non substitué, ou bien substitué par un groupe alkyle en $C_1$ à $C_4$, ou bien $R^3$ et $R^4$ forment, avec l'atome de carbone reliant ces restes, un noyau cycloalkyle (en $C_5$ à $C_{12}$) ou pipéridine, noyau non substitué ou comportant jusqu'à quatre substituants qui sont des groupes alkyles en $C_1$ à $C_4$,

$R^5$ représente, lorsque n vaut 1, un noyau pipéridine portant un à quatre groupes alkyles substituants en $C_1$ à $C_4$,

$R^5$ représente, lorsque n vaut 2, un reste cycloalkylène, dicycloalkylène, tricycloalkylène, bisméthylène monocycloalkylène, bisméthylènedicycloalkylène, bisméthylènetricycloalkylène, arylène, bisarylène alkyle, ces restes pouvant également être substitués par du brome ou du chlore ou par des restes alkyles en $C_1$ à $C_4$ , un reste

$$-R^6-O-(CH_2\underset{\displaystyle OZ}{\underset{|}{C}}HCH_2-O-R^6-O)_r$$

dans lequel r vaut 1 à 20 et chaque $R^6$ représente un reste alkylène en $C_2$ à $C_{12}$, cycloalkylène, dicycloalkylène, tricycloalkylène, bisméthylène monocycloalkylène, bisméthylènedicycloalkylène,bisméthylènetricycloalkylène, arylène, bisarylène alkyle, ces restes pouvant également être substitués par du brome ou du chlore ou par des restes alkyles en $C_1$ à $C_4$,

$R^5$ représente, lorsque n vaut 3, le reste d'une amine trifonctionnelle, le reste trifonctionnel d'un alcool aliphatique, qui contient encore d'autres groupes hydroxyles, le reste trifonctionnel d'une novolaque à base de phénol, de crésol, de bisphénol F ou de bisphénol A,

$R^5$ représente, lorsque n vaut 4, le reste tétrafonctionnel d'un alcool aliphatique ou d'une amine aliphatique ou d'une novolaque à base de phénol, de crésol, de bisphénol F ou de bisphénol A, ou le reste

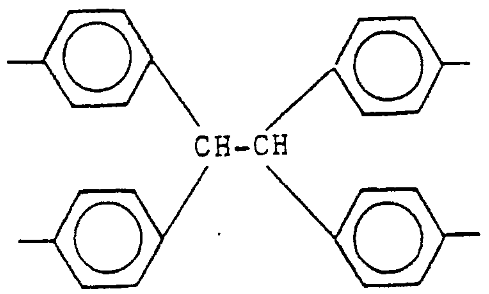

$R^5$ représente, lorsque n vaut plus de 4, un reste polyfonctionnel d'un polyol, d'une polyamine, d'une novolaque à base de phénol, de crésol, de bisphénol F ou de bisphénol A,

Y représente

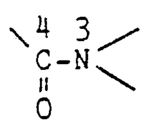 ou 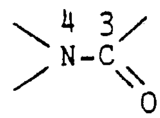

et prend les positions 3, 4 du noyau dans la formule (I),

X représente -O- ou

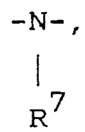

où

$R^7$ représente un atome d'hydrogène, un reste alkyle en $C_1$ à $C_{30}$, un noyau pyridine (substitué par un à quatre groupes alkyles en $C_1$ à $C_4$ ) ou un reste de formule (II)

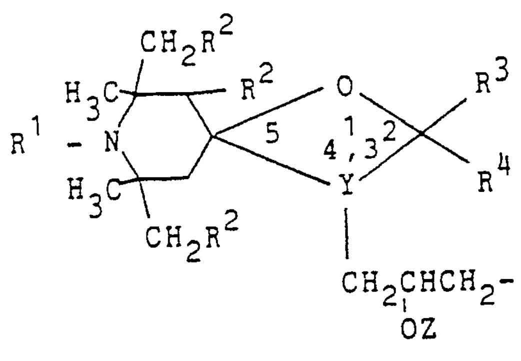 (II)

dans laquelle $R^1$, $R^2$, $R^3$, $R^4$ et Y ont les sens précités,

Z représente un atome d'hydrogène ou un reste de formule (III)

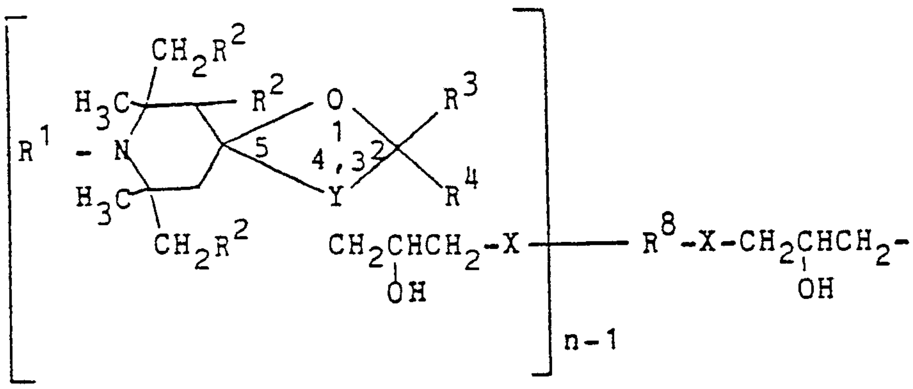

(III)

dans laquelle $R^1$, $R^2$, $R^3$, $R^4$, X, Y, Z et n ont les sens précités, ou bien un reste de formule (II) et

$R^8$ a le sens de $R^5$ et peut représenter encore :

lorsque n vaut 1, un atome d'hydrogène, un reste alkyyle en $C_1$ à $C_{30}$, aryle, aralkyle, alkylaryle ou un noyau pipéridine substitué par un à quatre groupes alkyles en $C_1$ à $C_4$, et

lorsque n vaut 2, $R^8$ représente un groupe alkylène en $C_2$ à $C_{18}$ ou un reste phénylène et X ne peut représenter alors que

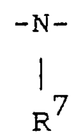

et

le groupe

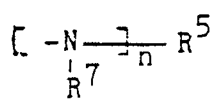

de la formule (I) peut représenter en outre

lorsque n vaut 2

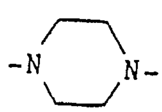

ou

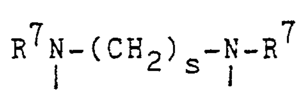

et

lorsque n vaut plus de 2, une polyamine de formule

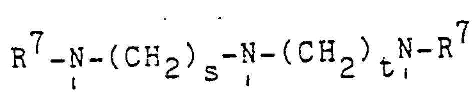

ou

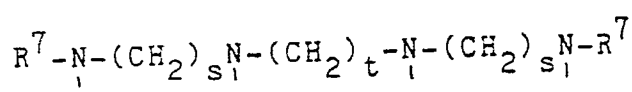

dans lesquelles $R^7$ a le sens précité, et s et t sont des nombres entiers valant 2 à 4.

**2.** Procédé pour préparer des composés de formule (I), procédé caractérisé en ce que

on fait réagir un diazaoxaspirodécane de formule (IVa) ou (IVb)

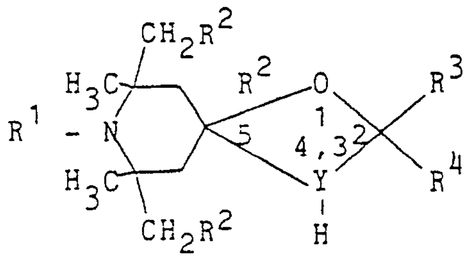

(IVa)

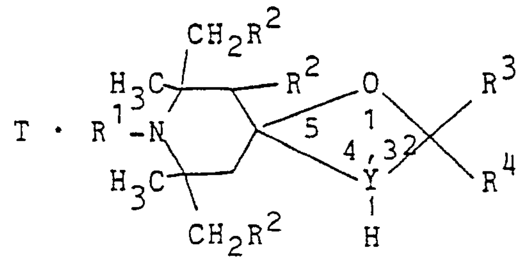

(IVb)

(formules dans lesquelles $R^1$, $R^2$, $R^3$, $R^4$ et Y ont le sens indiqué à la revendication 1, et T représente un acide minéral non oxydant ou un acide sulfonique ou phosphonique aliphatique ou aromatique, un acide mono-, di- ou polycarboxylique aliphatique ou un acide mono- ou dicarboxylique aromatique),

A) avec un époxyde de formule (Va) ou (Vb)

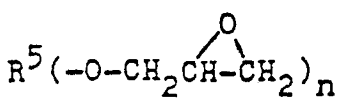

(Va)

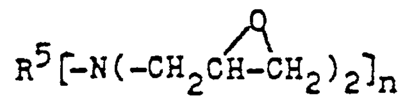

(Vb)

(dans lesquelles $R^5$ et n ont le sens indiqué à la revendication 1), dans un solvant inerte en présence d'une base et éventuellement d'un catalyseur de transfert de phase, entre 40 et 180 °C, ou bien

B) avec une épichlorhydrine comme en A) pour obtenir un composé de formule (VI)

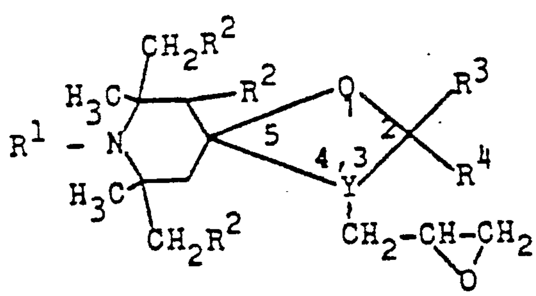

(VI)

et l'on fait réagir ce composé ensuite avec un alcool de formule (VII)

$R^5(OH)_n$ (VII)

ou avec une amine de formule (VIII)

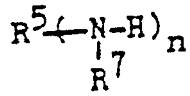

(VIII)

formules dans lesquelles $R^1$, $R^2$, $R^3$, $R^4$, $R^5$, $R^7$, Y et n ont les sens indiqués à la revendication 1) ou avec une polyamine de formule (IX)

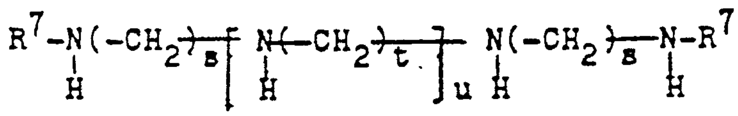 (IX)

(dans laquelle $R^7$, s et t ont les sens indiqués ci-dessus et u est un nombre entier valant 0 à 3

3. Utilisation des composés selon la revendication 1 pour stabiliser des polymères synthétiques.

4. Utilisation selon la revendication 3, caractérisée en ce que le polymère est une polyoléfine, un polyacrylate, un polyméthacrylate ou un homo- ou copolymère du styrène.

5. Utilisation selon la revendication 3, caractérisée en ce que le polymère constitue la partie solide d'une laque ou d'un vernis.

6. Procédé pour stabiliser des polymères synthétiques à l'encontre de l'influence nuisible de la lumière, procédé caractérisé en ce qu'on ajoute aux polymères, éventuellement en plus de substances connues jusqu'à présent et ayant un rôle de stabilisation, 0,01 à 5 parties en poids, par rapport au polymère, d'un stabilisant selon la revendication 1.

7. Polymères synthétiques stabilisés à l'encontre d'une décomposition sous l'effet de la lumière ultraviolette, ces polymères contenant 0,01 à 5 parties en poids, par rapport aux polymères, d'un stabilisant selon la revendication 1.

**Revendications pour l'Etat contractant suivant : AT**

1. Procédé pour préparer des composés de formule

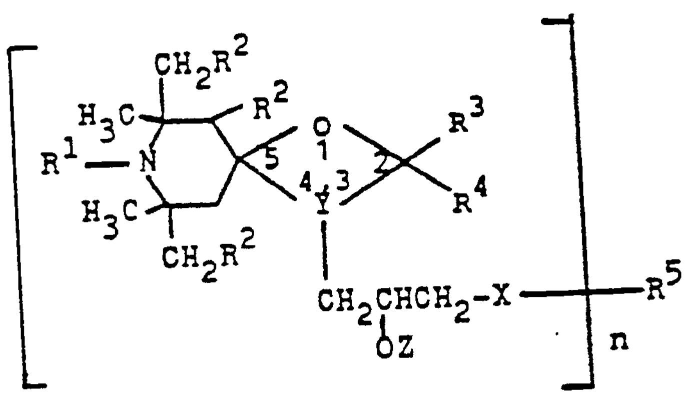 (I),

dans laquelle

n est un nombre entier valant 1 à 20 ;

$R^1$ représente un atome d'hydrogène ; un reste alkyle en $C_1$ à $C_4$, benzyle, allyle, alcanoyle en $C_2$ à $C_{30}$, alcénoyle en $C_2$ à $C_{20}$, aroyle en $C_7$ à $C_{11}$, arylalcanoyle en $C_8$ à $C_{14}$ ou alkylaryle en $C_8$ à $C_{20}$,

$R^2$ représente un atome d'hydrogène ou un reste alkyle en $C_1$ à $C_4$,

$R^3$ et $R^4$ sont identiques ou différents et représentent chacun un atome d'hydrogène, un reste alkyle en $C_1$ à $C_{18}$, un reste phényle (non substitué ou substitué par du chlore ou par un groupe alkyle en $C_1$ à $C_4$), un reste aralkyle non substitué, ou bien substitué par un groupe alkyle en $C_1$ à $C_4$, ou bien $R^3$ et $R^4$ forment, avec l'atome de carbone reliant ces restes, un noyau cycloalkyle (en $C_5$ à $C_{12}$) ou pipéridine, noyau non substitué ou comportant jusqu'à quatre substituants qui sont des groupes alkyles en $C_1$ à $C_4$,

$R^5$ représente, lorsque n vaut l, un noyau pipéridine portant un à quatre groupes alkyles substituants en $C_1$ à $C_4$,

$R^5$ représente, lorsque n vaut 2, un reste cycloalkylène, dicycloalkylène, tricycloalkylène, bisméthylène monocycloalkylène, bisméthylènedicycloalkylène, bisméthylènetricycloalkylène, arylène, bisaryléne alkyle, ces restes pouvant également être substitués par du brome ou du chlore ou par des restes alkyles en $C_1$ à $C_4$ , un reste

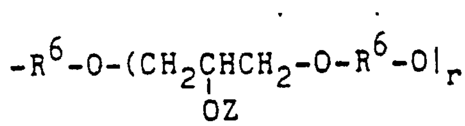

dans laquelle r vaut 1 à 20 et chaque $R^6$ représente un reste alkylène en $C_2$ à $C_{12}$, cycloalkylène, dicycloalkylène, tricycloalkylène, bisméthylène monocycloalkylène, bisméthylènedicycloalkylène,bisméthylènetricycloalkylène, arylène, bisarylène alkyle, ces restes pouvant également être substitués par du brome ou du chlore ou par des restes alkyles en $C_1$ à $C_4$,

$R^5$ représente, lorsque n vaut 3, le reste d'une amine trifonctionnelle, le reste trifonctionnel d'un alcool aliphatique, qui contient encore d'autres groupes hydroxyles, le reste trifonctionnel d'une novolaque à base de phénol, de crésol, de bisphénol F ou de bisphénol A,

$R^5$ représente, lorsque n vaut 4, le reste tétrafonctionnel d'un alcool aliphatique ou d'une amine aliphatique ou d'une novolaque à base de phénol, de crésol, de bisphénol F ou de bisphénol A, ou le reste

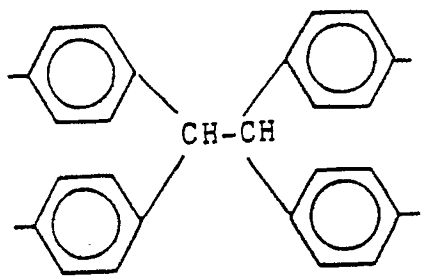

$R^5$ représente, lorsque n vaut plus de 4, un reste polyfonctionnel d'un polyol, d'une polyamine, d'une novolaque à base de phénol, de crésol, de bisphénol F ou de bisphénol A,

Y représente

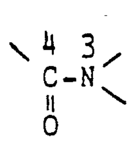 ou 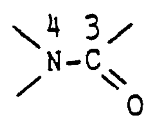

et prend les positions 3, 4 du noyau dans la formule (I),

X représente -O- ou

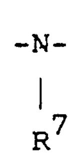

où

$R^7$ représente un atome d'hydrogène, un reste alkyle en $C_1$ à $C_{30}$, un noyau pyridine (substitué par un à quatre groupes alkyles en $C_1$ à $C_4$ ou un reste de formule (II)

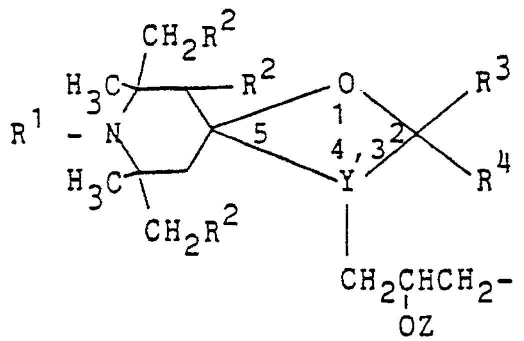

(II)

dans laquelle $R^1$, $R^2$, $R^3$, $R^4$ et Y ont les sens précités,

Z représente un atome d'hydrogène ou un reste de formule (III)

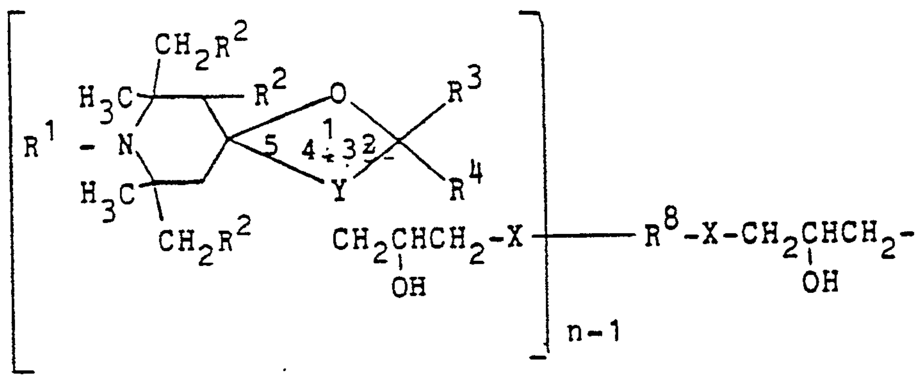

(III)

dans laquelle $R^1$, $R^2$, $R^3$, $R^4$, X, Y, Z et n ont les sens précités, ou bien un reste de formule (II) et

$R^8$ a le sens de $R^5$ et peut représenter encore :

lorsque n vaut 1, un atome d'hydrogène, un reste alkyle en $C_1$ à $C_{30}$, aryle, aralkyle, alkylaryle ou un noyau pipéridine substitué par un à quatre groupes alkyles en $C_1$ à $C_4$, et

lorsque n vaut 2, $R^8$ représente un groupe alkylène en $C_2$ à $C_{18}$ ou un reste phénylène et X ne peut représenter alors que

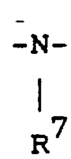

et

le groupe

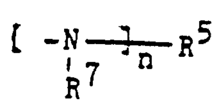

de la formule (I) peut représenter en outre

lorsque n vaut 2

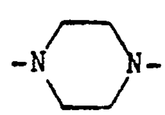 ou 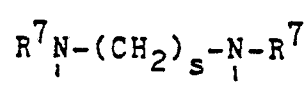

et

lorsque n vaut plus de 2, une polyamine de formule

$$R^7-N-(CH_2)_s-N-(CH_2)_t N-R^7$$

ou $$R^7-N-(CH_2)_s N-(CH_2)_t-N-(CH_2)_s N-R^7$$

dans lesquelles $R^7$ a le sens précité, et s et t sont des nombres entiers valant 2 à 4, caractérisé en ce que

on fait réagir un diazaoxaspirodécane de formule (IVa) ou (IVb)

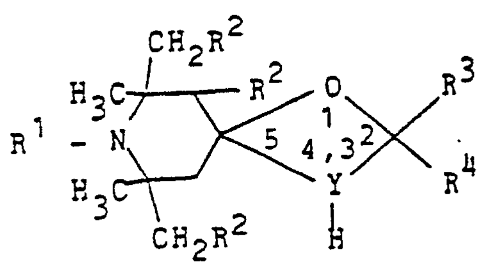

(IVa)

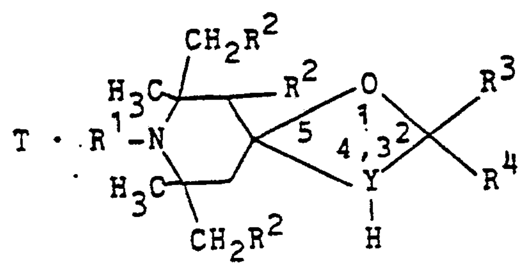

(IVb)

(formules dans lesquelles $R^1$, $R^2$, $R^3$, $R^4$ et Y ont le sens indiqué ci - dessus ) , et T représente un acide minéral non oxydant ou un acide sulfonique ou phosphonique aliphatique ou aromatique, un acide mono-, di- ou polycarboxylique aliphatique ou un acide mono- ou di- carboxylique aromatique),

A) avec un époxyde de formule (Va) ou (Vb)

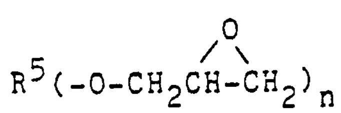

(Va)

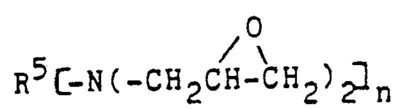

(Vb)

(dans lesquelles $R^5$ et n ont le sens indiqué ci - dessus ), dans un solvant inerte en présence d'une base et éventuellement d'un catalyseur de transfert de phase, entre 40 et 180 °C, ou bien

B) avec une épichlorhydrine comme en A) pour obtenir un composé de formule (VI)

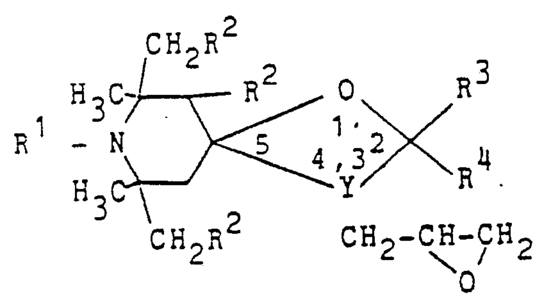

(VI)

et l'on fait réagir ce composé ensuite avec un alcool de formule (VII)

$R^5(-OH)_n$ (VII)

ou avec une amine de formule (VIII)

$$R^5(-\underset{R^7}{\underset{|}{N}}-H)_n \qquad (VIII)$$

(formules dans lesquelles $R^1$, $R^2$, $R^3$, $R^4$, $R^5$, $R^7$, Y et n ont les sens indiqués ci - dessus ) ou avec une polyamine de formule (IX)

$$R^7-\underset{H}{\underset{|}{N}}(-CH_2)_s\left[\underset{H}{\underset{|}{N}}(-CH_2)_t\right]_u\underset{H}{\underset{|}{N}}(-CH_2)_s\underset{H}{\underset{|}{N}}-R^7 \qquad (IX)$$

(dans laquelle $R^7$, s et t ont les sens indiqués ci-dessus et u est un nombre entier valont 0 à 3.

**2.** Utilisation des composés selon la revendication 1 pour stabiliser des polymères synthétiques.

**3.** Utilisation selon la revendication 2, caractérisée en ce que le polymère est une polyoléfine, un polyacrylate, un polyméthacrylate ou un homo- ou copolymère du styrène.

**4.** Utilisation selon la revendication 2, caractérisée en ce que le polymère constitue la partie solide d'une laque ou d'un vernis.

**5.** Procédé pour stabiliser des polymères synthétiques à l'encontre de l'influence nuisible de la lumière, procédé caractérisé en ce qu'on ajoute aux polymères, éventuellement en plus de substances connues jusqu'à présent et ayant un rôle de stabilisation, 0,01 à 5 parties en poids, par rapport au polymère, d'un stabilisant selon la revendication 1.

**6.** Polymères synthétiques stabilisés à l'encontre d'une décomposition sous l'effet de la lumière ultraviolette, ces polymères contenant 0,01 à 5 parties en poids, par rapport aux polymères, d'un stabilisant selon la revendication 1.